Europäisches Patentamt

European Patent Office

Office européen des brevets

(19)

(11) EP 0 752 475 A1

## (12) DEMANDE DE BREVET EUROPEEN

(43) Date de publication:
08.01.1997 Bulletin 1997/02

(51) Int Cl.6: C12N 15/55, C12N 9/16,
C12N 1/21, C12N 15/10

(21) Numéro de dépôt: 96401419.5

(22) Date de dépôt: 27.06.1996

(84) Etats contractants désignés:
AT CH DE FR GB IT LI SE

(30) Priorité: 29.06.1995 FR 9507833

(71) Demandeur: COMMISSARIAT A L'ENERGIE
ATOMIQUE
75015 Paris (FR)

(72) Inventeurs:
• Boulain, Jean-Claude
91120 Palaiseau (FR)

• Cattolico, Laurence
92330 Sceaux (FR)
• Ducancel, Frédéric
91160 Longjumeau (FR)
• Menez, André
78114 Magny les Hameaux (FR)

(74) Mandataire: Orès, Bernard et al
Cabinet ORES
6, Avenue de Messine
75008 Paris (FR)

(54) **Phosphatases alcalines bactériennes modifiées et leurs applications**

(57) Phosphatases alcalines modifiées d'origine bactérienne (BAP ou *Bacterial Alkaline Phosphatase*) constituées par une séquence de phosphatase alcaline bactérienne, dans laquelle au moins l'un des résidus d'amino-acide en position 329 ou en position 330 est substitué par un autre résidu d'amino-acide, lesquelles phosphatases alcalines bactériennes modifiées pré-sentent à la fois des propriétés enzymatiques significativement améliorées et une stabilité thermique élevée, et leurs applications notamment dans les dosages immuno-enzymatiques (réactifs et kits de diagnostic).

Procédé de sélection de mutants de phosphatases alcalines ayant des propriétés enzymatiques significa-tivement améliorées.

**LISTE DE SEQUENCES**

(1) INFORMATIONS GENERALES:

    (i) DEPOSANT:
        (A) NOM: COMMISSARIAT A L'ENERGIE ATOMIQUE
        (B) RUE: 31-33 RUE DE LA FEDERATION
        (C) VILLE: PARIS
        (E) PAYS: FRANCE
        (F) CODE POSTAL: 75015

    (ii) TITRE DE L' INVENTION: PHOSPHATASES ALCALINES BACTERIENNES
        MODIFIEES ET LEURS APPLICATIONS.

    (iii) NOMBRE DE SEQUENCES: 8

    (iv) FORME DECHIFFRABLE PAR ORDINATEUR:
        (A) TYPE DE SUPPORT: Floppy disk
        (B) ORDINATEUR: IBM PC compatible
        (C) SYSTEME D' EXPLOITATION: PC-DOS/MS-DOS
        (D) LOGICIEL: PatentIn Release #1.0, Version #1.30 (OEB)

    (vi) DONNEES DE LA DEMANDE ANTERIEURE:
        (A) NUMERO DE LA DEMANDE: FR 95 07833
        (B) DATE DE DEPOT: 29-JUN-1995

(2) INFORMATIONS POUR LA SEQ ID NO: 1:

    (i) CARACTERISTIQUES DE LA SEQUENCE:
        (A) LONGUEUR: 1443 paires de bases
        (B) TYPE: nucléotide
        (C) NOMBRE DE BRINS: simple
        (D) CONFIGURATION: linéaire

    (ii) TYPE DE MOLECULE: Autre acide nucléique
        (A) DESCRIPTION:   /desc = "modified DNA"

    (ix) CARACTERISTIQUE:
        (A) NOM/CLE: CDS
        (B) EMPLACEMENT:64..1440

    (xi) DESCRIPTION DE LA SEQUENCE: SEQ ID NO: 1:

```
GTGAAACAAA GCACTATTGC ACTGGCACTC TTACCGTTAC TGTTTACCCC TGTGACAAAA   60

GCC CGG ACA CCA GAA ATG CCC GTC GAC TTC AGT CGA CGA GCT CCC GGG   108
Arg Thr Pro Glu Met Pro Val Asp Phe Ser Arg Arg Ala Pro Gly
 1               5                   10                  15

GTT CTG GAA AAC CGG GCT GCT CAG GGC GAT ATT ACT GCA CCC GGC GGT   156
Val Leu Glu Asn Arg Ala Ala Gln Gly Asp Ile Thr Ala Pro Gly Gly
             20                  25                  30

GCT CGC CGT TTA ACG GGT GAT CAG ACT GCC GCT CTG CGT GAT TCT CTT   204
Ala Arg Arg Leu Thr Gly Asp Gln Thr Ala Ala Leu Arg Asp Ser Leu
             35                  40                  45

AGC GAT AAA CCT GCA AAA AAT ATT ATT TTG CTG ATT GGC GAT GGG ATG   252
Ser Asp Lys Pro Ala Lys Asn Ile Ile Leu Leu Ile Gly Asp Gly Met
             50                  55                  60
```

```
GGG GAC TCG GAA ATT ACT GCC GCA CGT AAT TAT GCC GAA GGT GCG GGC    300
Gly Asp Ser Glu Ile Thr Ala Ala Arg Asn Tyr Ala Glu Gly Ala Gly
        65                  70                  75

GGC TTT TTT AAA GGT ATA GAT GCC TTA CCG CTT ACC GGG CAA TAC ACT    348
Gly Phe Phe Lys Gly Ile Asp Ala Leu Pro Leu Thr Gly Gln Tyr Thr
    80                  85                  90                  95

CAC TAT GCG CTG AAT AAA AAA ACC GGC AAA CCG GAC TAC GTC ACC GAC    396
His Tyr Ala Leu Asn Lys Lys Thr Gly Lys Pro Asp Tyr Val Thr Asp
                100                 105                 110

TCG GCT GCA TCA GCA ACC GCC TGG TCA ACC GGT GTC AAA ACC TAT AAC    444
Ser Ala Ala Ser Ala Thr Ala Trp Ser Thr Gly Val Lys Thr Tyr Asn
            115                 120                 125

GGC GCG CTG GGC GTC GAT ATT CAC GAA AAA GAT CAC CCA ACG ATT CTG    492
Gly Ala Leu Gly Val Asp Ile His Glu Lys Asp His Pro Thr Ile Leu
        130                 135                 140

GAA ATG GCA AAA GCC GCA GGT CTG GCG ACC GGT AAC GTT TCT ACC GCA    540
Glu Met Ala Lys Ala Ala Gly Leu Ala Thr Gly Asn Val Ser Thr Ala
    145                 150                 155

GAG TTG CAG CAC GCC ACG CCC GCT GCG CTG GTG GCA CAT GTG ACC TCG    588
Glu Leu Gln His Ala Thr Pro Ala Ala Leu Val Ala His Val Thr Ser
160                 165                 170                 175

CGC AAA TGC TAC GGT CCG AGC GCG ACC AGT GAA AAA TGT CCG GGT AAC    636
Arg Lys Cys Tyr Gly Pro Ser Ala Thr Ser Glu Lys Cys Pro Gly Asn
                180                 185                 190

GCT CTG GAA AAA GGC GGA AAA GGA TCG ATT ACC GAA CAG CTG CTT AAC    684
Ala Leu Glu Lys Gly Gly Lys Gly Ser Ile Thr Glu Gln Leu Leu Asn
            195                 200                 205

GCT CGT GCC GAC GTT ACG CTT GGC GGC GGC GCA AAA ACC TTT GCT GAA    732
Ala Arg Ala Asp Val Thr Leu Gly Gly Gly Ala Lys Thr Phe Ala Glu
        210                 215                 220

ACG GCA ACC GCT GGT GAA TGG CAG GGA AAA ACG CTG CGT GAA CAG GCA    780
Thr Ala Thr Ala Gly Glu Trp Gln Gly Lys Thr Leu Arg Glu Gln Ala
    225                 230                 235

CAG GCG CGT GGT TAT CAG TTG GTG AGC GAT GCT GCC TCA CTG AAT TCG    828
Gln Ala Arg Gly Tyr Gln Leu Val Ser Asp Ala Ala Ser Leu Asn Ser
240                 245                 250                 255

GTG ACG GAA GCG AAT CAG CAA AAA CCC CTG CTT GGC CTG TTT GCT GAC    876
Val Thr Glu Ala Asn Gln Gln Lys Pro Leu Leu Gly Leu Phe Ala Asp
                260                 265                 270

GGC AAT ATG CCA GTG CGC TGG CTA GGA CCG AAA GCA ACG TAC CAT GGC    924
Gly Asn Met Pro Val Arg Trp Leu Gly Pro Lys Ala Thr Tyr His Gly
            275                 280                 285

AAT ATC GAT AAG CCC GCA GTC ACC TGT ACG CCA AAT CCG CAA CGT AAT    972
Asn Ile Asp Lys Pro Ala Val Thr Cys Thr Pro Asn Pro Gln Arg Asn
        290                 295                 300

GAC AGT GTA CCA ACC CTG GCG CAG ATG ACC GAC AAA GCC ATT GAA TTG    1020
Asp Ser Val Pro Thr Leu Ala Gln Met Thr Asp Lys Ala Ile Glu Leu
        305                 310                 315
```

```
TTG AGT AAA AAT GAG AAA GGC TTT TTC CTG CAA GTT GAA GGT GCG TCA   1068
Leu Ser Lys Asn Glu Lys Gly Phe Phe Leu Gln Val Glu Gly Ala Ser
320               325               330                   335

ATC GAT CAC CAG AAT CAT GCT GCG AAT CCT TGT GGG CAA ATT GGC GAG   1116
Ile Asp His Gln Asn His Ala Ala Asn Pro Cys Gly Gln Ile Gly Glu
                  340               345                   350

ACG GTC GAT CTC GAT GAA GCC GTA CAA CGG GCG CTG GAA TTC GCT AAA   1164
Thr Val Asp Leu Asp Glu Ala Val Gln Arg Ala Leu Glu Phe Ala Lys
              355               360               365

AAG GAG GGT AAC ACG CTG GTC ATA GTC ACC GCT GAT CAC GCC CAC GCC   1212
Lys Glu Gly Asn Thr Leu Val Ile Val Thr Ala Asp His Ala His Ala
              370               375               380

AGC CAG ATT GTT GCG CCG GAT ACC AAA GCT CCG GGC CTC ACC CAG GCG   1260
Ser Gln Ile Val Ala Pro Asp Thr Lys Ala Pro Gly Leu Thr Gln Ala
          385               390               395

CTA AAT ACC AAA GAT GGC GCA GTG ATG GTG ATG AGT TAC GGG AAC TCC   1308
Leu Asn Thr Lys Asp Gly Ala Val Met Val Met Ser Tyr Gly Asn Ser
400               405               410                   415

GAA GAG GAT TCA CAA GAA CAT ACC GGC AGT CAG TTG CGT ATT GCG GCG   1356
Glu Glu Asp Ser Gln Glu His Thr Gly Ser Gln Leu Arg Ile Ala Ala
                  420               425               430

TAT GGC CCG CAT GCC GCC AAT GTT GTT GGA CTG ACC GAC CAG ACC GAT   1404
Tyr Gly Pro His Ala Ala Asn Val Val Gly Leu Thr Asp Gln Thr Asp
              435               440               445

CTC TTC TAC ACC ATG AAA GCC GCT CTG GGG CTG AAA TAA               1443
Leu Phe Tyr Thr Met Lys Ala Ala Leu Gly Leu Lys
          450               455
```

(2)  INFORMATIONS POUR LA SEQ ID NO: 2:

      (i) CARACTERISTIQUES DE LA SEQUENCE:
         (A) LONGUEUR: 459 acides aminés
         (B) TYPE: acide aminé
         (D) CONFIGURATION: linéaire

      (ii) TYPE DE MOLECULE: protéine
      (xi) DESCRIPTION DE LA SEQUENCE: SEQ ID NO: 2:

```
Arg Thr Pro Glu Met Pro Val Asp Phe Ser Arg Arg Ala Pro Gly Val
1               5               10                  15

Leu Glu Asn Arg Ala Ala Gln Gly Asp Ile Thr Ala Pro Gly Gly Ala
              20              25              30

Arg Arg Leu Thr Gly Asp Gln Thr Ala Ala Leu Arg Asp Ser Leu Ser
          35              40                  45

Asp Lys Pro Ala Lys Asn Ile Ile Leu Leu Ile Gly Asp Gly Met Gly
      50              55              60

Asp Ser Glu Ile Thr Ala Ala Arg Asn Tyr Ala Glu Gly Ala Gly Gly
65              70              75                  80

Phe Phe Lys Gly Ile Asp Ala Leu Pro Leu Thr Gly Gln Tyr Thr His
              85              90                  95
```

```
Tyr Ala Leu Asn Lys Lys Thr Gly Lys Pro Asp Tyr Val Thr Asp Ser
            100                 105                 110

Ala Ala Ser Ala Thr Ala Trp Ser Thr Gly Val Lys Thr Tyr Asn Gly
            115                 120                 125

Ala Leu Gly Val Asp Ile His Glu Lys Asp His Pro Thr Ile Leu Glu
        130                 135                 140

Met Ala Lys Ala Ala Gly Leu Ala Thr Gly Asn Val Ser Thr Ala Glu
145                 150                 155                 160

Leu Gln His Ala Thr Pro Ala Ala Leu Val Ala His Val Thr Ser Arg
                165                 170                 175

Lys Cys Tyr Gly Pro Ser Ala Thr Ser Glu Lys Cys Pro Gly Asn Ala
            180                 185                 190

Leu Glu Lys Gly Gly Lys Gly Ser Ile Thr Glu Gln Leu Leu Asn Ala
        195                 200                 205

Arg Ala Asp Val Thr Leu Gly Gly Gly Ala Lys Thr Phe Ala Glu Thr
    210                 215                 220

Ala Thr Ala Gly Glu Trp Gln Gly Lys Thr Leu Arg Glu Gln Ala Gln
225                 230                 235                 240

Ala Arg Gly Tyr Gln Leu Val Ser Asp Ala Ala Ser Leu Asn Ser Val
            245                 250                 255

Thr Glu Ala Asn Gln Gln Lys Pro Leu Leu Gly Leu Phe Ala Asp Gly
            260                 265                 270

Asn Met Pro Val Arg Trp Leu Gly Pro Lys Ala Thr Tyr His Gly Asn
        275                 280                 285

Ile Asp Lys Pro Ala Val Thr Cys Thr Pro Asn Pro Gln Arg Asn Asp
    290                 295                 300

Ser Val Pro Thr Leu Ala Gln Met Thr Asp Lys Ala Ile Glu Leu Leu
305                 310                 315                 320

Ser Lys Asn Glu Lys Gly Phe Phe Leu Gln Val Glu Gly Ala Ser Ile
            325                 330                 335

Asp His Gln Asn His Ala Ala Asn Pro Cys Gly Gln Ile Gly Glu Thr
        340                 345                 350

Val Asp Leu Asp Glu Ala Val Gln Arg Ala Leu Glu Phe Ala Lys Lys
        355                 360                 365

Glu Gly Asn Thr Leu Val Ile Val Thr Ala Asp His Ala His Ala Ser
    370                 375                 380

Gln Ile Val Ala Pro Asp Thr Lys Ala Pro Gly Leu Thr Gln Ala Leu
385                 390                 395                 400

Asn Thr Lys Asp Gly Ala Val Met Val Met Ser Tyr Gly Asn Ser Glu
            405                 410                 415

Glu Asp Ser Gln Glu His Thr Gly Ser Gln Leu Arg Ile Ala Ala Tyr
        420                 425                 430

Gly Pro His Ala Ala Asn Val Val Gly Leu Thr Asp Gln Thr Asp Leu
        435                 440                 445
```

21

```
Phe Tyr Thr Met Lys Ala Ala Leu Gly Leu Lys
    450                 455
```

(2) INFORMATIONS POUR LA SEQ ID NO: 3:

    (i) CARACTERISTIQUES DE LA SEQUENCE:
        (A) LONGUEUR: 38 paires de bases
        (B) TYPE: nucléotide
        (C) NOMBRE DE BRINS: simple
        (D) CONFIGURATION: linéaire

    (ii) TYPE DE MOLECULE: Autre acide nucléique
        (A) DESCRIPTION:   /desc = "oligonucleotide"

    (xi) DESCRIPTION DE LA SEQUENCE: SEQ ID NO: 3:

TGTACAAATA CATTAAAGGA TCCAAACAAA GCGACTAT          38

(2) INFORMATIONS POUR LA SEQ ID NO: 4:

    (i) CARACTERISTIQUES DE LA SEQUENCE:
        (A) LONGUEUR: 34 paires de bases
        (B) TYPE: nucléotide
        (C) NOMBRE DE BRINS: simple
        (D) CONFIGURATION: linéaire

    (ii) TYPE DE MOLECULE: Autre acide nucléique
        (A) DESCRIPTION:   /desc = "oligonucleotide"

    (xi) DESCRIPTION DE LA SEQUENCE: SEQ ID NO: 4:

GATTCGCAGC ATGATGACCG TGATCGATTG ACGC          34

(2) INFORMATIONS POUR LA SEQ ID NO: 5:

    (i) CARACTERISTIQUES DE LA SEQUENCE:
        (A) LONGUEUR: 24 paires de bases
        (B) TYPE: nucléotide
        (C) NOMBRE DE BRINS: simple
        (D) CONFIGURATION: linéaire

    (ii) TYPE DE MOLECULE: Autre acide nucléique
        (A) DESCRIPTION:   /desc = "oligonucleotide"

    (xi) DESCRIPTION DE LA SEQUENCE: SEQ ID NO: 5:

AACAACATTG GCGGCATGCG GGCC          24

(2) INFORMATIONS POUR LA SEQ ID NO: 6:

    (i) CARACTERISTIQUES DE LA SEQUENCE:
        (A) LONGUEUR: 24 paires de bases
        (B) TYPE: nucléotide
        (C) NOMBRE DE BRINS: simple
        (D) CONFIGURATION: linéaire

    (ii) TYPE DE MOLECULE: Autre acide nucléique
        (A) DESCRIPTION:   /desc = "oligonucleotide"

    (xi) DESCRIPTION DE LA SEQUENCE: SEQ ID NO: 6:

GACTTCAGTC GACGAGCTCC CGGG          24

```
(2)  INFORMATIONS POUR LA SEQ ID NO: 7:

     (i)  CARACTERISTIQUES DE LA SEQUENCE:
          (A)  LONGUEUR: 30 paires de bases
          (B)  TYPE: nucléotide
          (C)  NOMBRE DE BRINS: simple
          (D)  CONFIGURATION: linéaire

     (ii) TYPE DE MOLECULE: Autre acide nucléique
          (A)  DESCRIPTION:  /desc = "oligonucleotide"

     (xi) DESCRIPTION DE LA SEQUENCE: SEQ ID NO: 7:

GAAATGCCCG TCGACAGGAT ATGTTTTAAC                              30

(2)  INFORMATIONS POUR LA SEQ ID NO: 8:

     (i)  CARACTERISTIQUES DE LA SEQUENCE:
          (A)  LONGUEUR: 30 paires de bases
          (B)  TYPE: nucléotide
          (C)  NOMBRE DE BRINS: simple
          (D)  CONFIGURATION: linéaire

     (ii) TYPE DE MOLECULE: Autre acide nucléique
          (A)  DESCRIPTION:  /desc = "oligonucleotide"

     (xi) DESCRIPTION DE LA SEQUENCE: SEQ ID NO: 8:

GAACCCCGGG AGCTCCATTG TTGCAGACCT                              30
```

**Description**

La présente invention est relative à des phosphatases alcalines bactériennes (BAP ou *Bacterial Alkaline Phosphatase*) modifiées présentant à la fois des propriétés enzymatiques significativement améliorées et une stabilité thermique élevée, et à leurs applications notamment dans les dosages immuno-enzymatiques (réactifs et kits de diagnostic).

La présente invention est également relative à un procédé d'obtention de mutants ayant des propriétés enzymatiques significativement améliorées.

La phosphatase alcaline est une phosphomonoestérase non spécifique. Cette métalloenzyme dimérique comprend deux chaînes polypeptidiques identiques contenant chacune deux atomes de zinc et un atome de magnésium, situés au coeur du site catalytique.

Le mécanisme catalytique comprend plusieurs étapes dont la formation d'un complexe intermédiaire covalent phosphoryl-enzyme (E-$P_i$) avec la sérine 102 de la protéine, conformément à la réaction suivante :

$$E + R_1OP_i \underset{k_{-1}}{\overset{k_1}{<\longrightarrow>}} E.R_1OP_i \underset{k_{-2}(R_1OH)}{\overset{k_2}{<\longrightarrow>}} E\text{-}P_i \underset{k_{-3}}{\overset{k_3}{<\longrightarrow>}} E.P_i \underset{k_{-4}}{\overset{k_4}{<\longrightarrow>}} E + P_i \qquad (1)$$

La constante catalytique de la réaction ($k_{cat}$) dépend de l'étape limitante du processus. A pH acide, le facteur limitant est la rupture de la liaison covalente E-$P_i$ ; à pH alcalin, c'est la dissociation du complexe non-covalent E.$P_i$ qui est l'étape limitante (BUTLER-RANSOHOFF et al., J. Org. Chem., 1992, 57, 142-145).

En présence d'un accepteur de phosphate ($R_2OH$), tel que l'éthanolamine ou le Tris, l'enzyme catalyse une réaction de transphosphorylation avec le transfert du groupe phosphoryl à l'alcool, conformément à la formule (2) ci-après :

$$E\text{-}P_i \underset{k_{-5}}{\overset{k_5(R_2OH)}{<\longrightarrow>}} E + R_2OP \qquad (2)$$

L'efficacité catalytique est appréciée par le rapport $k_{cat}/K_M$, qui tient compte à la fois de l'activité hydrolytique et de l'affinité pour le substrat, de l'enzyme.

On trouve la phosphatase alcaline chez tous les organismes, des bactéries aux mammifères.

Les enzymes présentes chez les mammifères possèdent les activités spécifiques les plus élevées, c'est pourquoi la phosphatase alcaline d'intestin de veau (CIP = *Calf Intestine Phosphatase*) est habituellement choisie pour construire les réactifs immuno-enzymatiques, utilisés dans les tests de diagnostic *in vitro*. La CIP est cependant difficile à purifier de façon reproductible et présente une faible stabilité thermique.

La phosphatase alcaline bactérienne (BAP), qui présente une activité enzymatique significativement inférieure à celle de la CIP, est produite en grande quantité par *E. coli ;* elle peut être purifiée aisément et possède une stabilité thermique exceptionnelle. En outre, elle peut être utilisée pour la construction de réactifs immuno-enzymatiques par des techniques de fusion génétique.

Différentes équipes ont, en conséquence, tenté d'améliorer les propriétés catalytiques de l'enzyme bactérienne, afin de la substituer à la CIP dans la fabrication des réactifs de dosage.

L'introduction de mutations ponctuelles dans le site actif de la BAP a permis d'obtenir des molécules possédant une activité enzymatique augmentée par rapport à l'enzyme de type sauvage :

CHAIDAROGLOU et KANTROWITZ (*Protein Engineering,* 1989, 3, 2, 127-132) ont décrit un mutant de la BAP d'*E. coli,* dans lequel l'acide aspartique en position 101 est remplacé par de l'alanine (mutant dénommé D101A). Un tel mutant présente, à pH 9,4 et en présence d'accepteur de phosphate, une activité catalytique trois fois supérieure à celle de l'enzyme de type sauvage, mais une nette diminution de la stabilité à la chaleur.

Les études de stabilité thermique ont été réalisées dans des conditions différentes de celles communément admises ; toutefois, elles montrent clairement que le mutant a une stabilité thermique inférieure à celle de l'enzyme sauvage.

La Demande de Brevet européen 0441252, au nom de Abbott Laboratories, décrit des phosphatases alcalines ayant une activité spécifique améliorée (activité catalytique trente six fois supérieure à celle de l'enzyme de type sauvage), à pH 10, en présence de faibles concentrations soit de Tris 0,05 M, soit de diéthanolamine 0,05 M, mais qui présentent une stabilité thermique diminuée, en vue de leur utilisation comme réactifs. Les mutations décrites dans cette Demande se situent soit à une distance d'environ 20 Å du site actif de l'enzyme, soit à une distance d'environ 10 Å du site actif de l'enzyme, soit dans le site actif et incluent :

(i) les mutations ne concernant qu'un seul amino-acide, telles que :

- la substitution de Thr$^{100}$ par Val ou Ile (mutants T100V ou T100I)
- la substitution de Lys$^{328}$ par Arg (mutant L328R)
- la substitution de Val$^{99}$ par Ala, (mutant V99A)
- la substitution de Ala$^{103}$ par Asp ou Cys (mutants A103D ou A103C)
- la substitution de Thr$^{107}$ par Val (mutant T107V)
- la substitution de Asp$^{101}$ par Ser (mutant D101S)

(ii) les mutations faisant intervenir deux amino-acides, telles que :

- la substitution de Val$^{99}$ par Ala et de Lys$^{328}$ par Arg (mutant V99A et K328R)
- la substitution de Val$^{377}$ par Ala et de Ser$^{415}$ par Gly (mutant V377A et S415G).

La Demande Internationale PCT WO 94/01531, également au nom de Abbott Laboratories et qui reprend en substance le contenu de la Demande européenne précitée, décrit en outre la substitution de Asp$^{153}$ par Gly (mutant D153G).

De manière générale, pour mesurer une activité catalytique, il est nécessaire de se placer pour une enzyme donnée, dans les conditions optimales de son activité ; or, dans ces Demandes Abbott, l'activité de l'enzyme sauvage et des mutants est comparée dans les mêmes conditions, en l'occurrence dans les conditions optimales des mutants.

En conséquence, dans le cas des Demandes Abbott, l'augmentation de l'activité catalytique d'un facteur 36, résulte de la comparaison de l'activité catalytique du mutant placé dans les conditions optimales de fonctionnement, avec l'activité catalytique de l'enzyme sauvage dans les mêmes conditions. Si l'on compare ces activités dans les conditions optimales de fonctionnement de chacune des enzymes, le facteur d'accroissement des propriétés catalytiques se limite à 18.

En outre, aucun de ces mutants (qui diffèrent de la BAP de type sauvage par seulement un ou deux résidus au plus) ne parvient à procurer à la phosphatase alcaline modifiée à la fois une activité enzymatique équivalente à celle de l'enzyme de mammifère correspondante et une stabilité thermique significativement supérieure à celle de l'enzyme de mammifère et notamment la stabilité thermique de l'enzyme bactérienne de départ.

D'autres travaux ont été réalisés, dont l'objectif était de mieux comprendre le mécanisme catalytique de l'enzyme. Plus précisément, les Auteurs de ces différents articles se sont attachés à déterminer les causes moléculaires à l'origine des différences observées entre la BAP et les enzymes de mammifères : activité enzymatique 20 à 30 fois plus élevée des enzymes de mammifères, déplacement de l'activité optimale vers les pH élevés, addition de magnésium nécessaire à l'obtention d'une activité maximale. Dans la région du site actif, il existe deux différences notables entre les enzymes bactériennes et les enzymes de mammifères. Dans les enzymes bactériennes, les positions 153 et 328 sont occupées par les résidus Asp et Lys respectivement, tandis qu'un résidu His est présent à ces deux positions dans les enzymes de mammifères. Afin d'évaluer l'importance de ces différences sur l'activité enzymatique, des résidus His ont été introduit sur ces deux positions dans l'enzyme bactérienne (XU et KANTROWITZ, Biochemistry, 1991, **30**, 7789-7796; JANEWAY et al., Biochemistry, 1993, **32**, 1601-1609).

XU et KANTROWITZ décrivent en particulier la substitution de la lysine en position 328, par une histidine, et les propriétés du mutant obtenu (K328H). En présence d'un accepteur de phosphate, le mutant K328H a une activité comparable à celle de l'enzyme sauvage; par contre, à pH 8 et en l'absence d'accepteur de phosphate, ce mutant présente une diminution significative de l'activité catalytique, comparée à celle de l'enzyme sauvage. Ces résultats suggèrent que ces mutations entraînent une inhibition de l'activité hydrolytique, accompagnée d'une augmentation de l'activité de transphosphorylation. En outre, ce mutant a une affinité réduite pour le phosphate. En résumé, une telle mutation entraîne : - un déplacement de l'activité enzymatique optimale vers pH 10, - une augmentation de l'activité spécifique (surtout activité transférase) et - une baisse d'affinité pour le substrat et pour le phosphate inorganique P$_i$, à pH 10.

Cette baisse d'affinité pour P$_i$ est sans doute liée à la suppression de la liaison au P$_i$, par l'intermédiaire d'une molécule d'eau, qui accélère l'étape limitante de la libération du P$_i$ par l'enzyme.

MATLIN et al. (Biochemistry, 1992, **31**, 8196-8200) ont étudié d'autres mutations au niveau de la position 153 de la phosphatase alcaline d'*E. coli* (remplacement de l'acide aspartique par de l'alanine: D153A ou par de l'asparagine : D153N) et ont également montré que la présence de magnésium est indispensable à l'activité de ces mutants. En outre, alors que le mutant D153N présente des paramètres cinétiques similaires à ceux de l'enzyme sauvage, le mutant D153A entraîne une augmentation d'un facteur 6,3 du k$_{cat}$, une augmentation d'un facteur 13,7 du rapport k$_{cat}$/K$_M$ (Tris 50 mM, pH 8) et une augmentation d'un facteur 159 du K$_i$ pour le P$_i$ (Tris 1 M, pH 8). De plus l'activité de ce mutant augmente d'un facteur 25, lorsque le pH est augmenté de 7 à 9.

L'importance de la présence de magnésium pour avoir effectivement une activité catalytique, tant pour le mutant D153A que pour un mutant D153H a été mise en évidence par MURPHY et al. (J. Biol. Chem., 1993, **268**, 29, 21497-21500), qui montrent que la mutation D153H entraîne la conversion du site de liaison au magnésium en un site de liaison au zinc.

JANEWAY et al. ont également exploré le rôle des résidus en position 153 et 328, de la phosphatase alcaline d'*E. coli.* Les mutants D153H (remplacement de l'acide aspartique en position 153 par une histidine), K328H (remplacement de la lysine en position 328 par une histidine) et D153H/K328H ont été plus particulièrement étudiés ainsi que les interactions, au niveau du site actif de la phosphatase alcaline, faisant intervenir de l'eau.

Il ressort de cet article que :

- le mutant D153H présente un déplacement de l'activité enzymatique optimale vers pH 10 et une baisse d'affinité pour le magnésium ; en présence de magnésium additionnel, l'activité catalytique est restaurée et est même supérieure à celle de l'enzyme sauvage.

Cette modification d'activité est sans doute due au fait que D153, dans l'enzyme sauvage, lie le magnésium par l'intermédiaire de deux molécules d'eau ; sa suppression déstabilise le magnésium qui est remplacé par un zinc (MURPHY et al., 1993, précité) et donne une forme inactive de l'enzyme.

L'augmentation de l'activité catalytique en présence de magnésium n'est cependant pas claire ; elle serait due à un effet indirect sur la K328, qui n'a plus de lien avec la D153.

Aussi bien MATLIN et al. (référence précitée) que JANEWAY et al. (référence précitée) montrent le rôle essentiel du magnésium dans l'évaluation de l'activité enzymatique de la phosphatase alcaline.

Le mutant K328H/D153H présente le même comportement que le mutant D153H, par rapport au magnésium ; en outre, il présente un KM diminué et un $k_{cat}$ augmenté.

Ceci montre la complexité de l'action et la difficulté d'évaluation de l'intérêt d'un mutant.

MURPHY et al. (Molecular Microbiology, 1994, **12**, 3, 351-357) résument également les propriétés des mutants D153H, K328H et D153H/K328H et aboutissent aux mêmes conclusions que les différents Auteurs précités.

En conclusion, les mutants K328H ou D153H présentent une augmentation modérée de l'activité spécifique. Le double mutant (D153H/K328H) est plus actif que les simples mutants, mais sa stabilité thermique est moins élevée que celle de l'enzyme sauvage et la présence de magnésium est indispensable à l'expression d'une activité élevée ; en outre, ce double mutant D153H/K328H présente une augmentation du $K_M$ d'un facteur supérieur à 30, ce qui entraîne une diminution du rapport $k_{cat}/K_M$ (faible affinité pour le substrat).

Bien que les résidus His en position 153 et/ou 328 participent aux performances élevées de la CIP, leur seule présence ne suffit pas à conférer à la BAP les caractéristiques recherchées, pour obtenir un outil de diagnostic efficace et stable, c'est-à-dire ayant à la fois :

- une activité catalytique (hydrolyse) de l'ordre de celle de la phosphatase alcaline de mammifère (CIP notamment),
- une grande affinité pour le substrat phosphoré, et
- une grande stabilité thermique, c'est-à-dire de l'ordre de celle de la BAP sauvage.

En conséquence, la Demanderesse s'est donné pour but de pourvoir à des phosphatases alcalines bactériennes modifiées, qui répondent mieux aux besoins de la pratique que les phosphatases alcalines de l'Art antérieur.

La présente invention a pour objet une phosphatase alcaline modifiée d'origine bactérienne, caractérisée en ce qu'elle est constituée par une séquence de phosphatase alcaline bactérienne (BAP), dans laquelle au moins l'un des résidus d'amino-acide en position 329 ou en position 330 est substitué par un autre résidu d'amino-acide, laquelle phosphatase alcaline modifiée présente une activité catalytique et une affinité pour le substrat significativement augmentées (activité enzymatique améliorée) par rapport auxdites activités de la phosphatase alcaline bactérienne sauvage correspondante et une stabilité thermique, de l'ordre de celle de ladite phosphatase alcaline bactérienne sauvage.

On entend par résidu d'amino-acide, au sens de la présente invention, n'importe quel résidu d'amino-acide naturel et notamment: Ala, Cys, Asp, Glu, Phe, Gly, His, Ile, Lys, Leu, Met, Asn, Pro, Gln, Arg, Ser, Thr, Val, Trp ou Tyr.

On entend par phosphatase alcaline d'origine bactérienne modifiée (BAPm), une phosphatase alcaline biologiquement active, dont les mutations ne correspondent pas nécessairement à des résidus d'amino-acide de phosphatase alcaline de mammifère (CIP, par exemple), laquelle BAPm peut notamment être obtenue, par mutagénèse aléatoire ou dirigée, à partir d'une phosphatase alcaline chimère, c'est-à-dire d'une phosphatase alcaline bactérienne comprenant au moins deux résidus d'amino-acide d'une phosphatase alcaline de mammifère, et notamment de la phosphatase alcaline d'intestin de veau (CIP) et biologiquement inactive.

On entend par enzyme présentant une activité enzymatique améliorée une enzyme qui présente un $k_{cat}$ augmenté et/ou un $K_M$ diminué par rapport à une enzyme non modifiée.

Selon un mode de réalisation avantageux de ladite phosphatase alcaline bactérienne modifiée, elle comprend en outre une substitution au niveau du résidu d'amino-acide 153 et/ou du résidu d'amino-acide 328.

Selon un autre mode de réalisation avantageux de ladite phosphatase alcaline bactérienne modifiée, la substitution au niveau de la position 330 est de préférence la substitution d'un acide aspartique (Asp[330] ou D) par une asparagine (Asn ou N), une alanine (Ala ou A) ou une leucine (Leu ou L).

Selon un autre mode de réalisation avantageux de ladite phosphatase alcaline modifiée, la substitution au niveau de la position 329 est de préférence la substitution d'une glutamine (Gln[329] ou Q) par une alanine (Ala ou A).

Conformément à l'invention, ladite phosphatase alcaline modifiée comprend en outre une histidine (His ou H) en position 153 à la place d'un acide aspartique (Asp[153]) et/ou une histidine en position 328 à la place d'une lysine (Lys ou K).

Conformément à l'invention, les mutants préférés sont sélectionnés parmi :

le mutant D330N, le mutant D153H/D330N, le mutant K328H/D330N, le mutant D153H/K328H/D330N, le mutant D330A, le mutant D330L, le mutant D153H/D330A, le mutant D153H/D330L, le mutant K328H/D330A, le mutant K328H/D330L, le mutant D153H/K328H/D330A, le mutant D153H/K328H/D330L, le mutant Q329A, le mutant D153H/Q329A, le mutant K328H/Q329A, le mutant D153H/K328H/Q329A.

Egalement conformément à l'invention, la séquence de phosphatase alcaline bactérienne est de préférence issue d'*Escherichia coli* ou de *Bacillus subtilis.*

Selon un autre mode de réalisation avantageux de ladite phosphatase alcaline bactérienne modifiée, elle comprend, outre au moins l'une des substitutions telles que définies ci-dessus, au moins un amino-acide supplémentaire, inséré entre les amino-acides +6 et +7 de ladite phosphatase alcaline bactérienne.

Parmi ces séquences modifiées, on peut citer la SEQ ID N°2 :

```
                          6                                    7
Arg Thr Pro Glu Met Pro Val Asp Phe Ser Arg Arg Ala Pro Gly Val
 1               5                  10                   15

Leu Glu Asn Arg Ala Ala Gln Gly Asp Ile Thr Ala Pro Gly Gly Ala
             20                  25                   30

Arg Arg Leu Thr Gly Asp Gln Thr Ala Ala Leu Arg Asp Ser Leu Ser
         35                  40                   45

Asp Lys Pro Ala Lys Asn Ile Ile Leu Leu Ile Gly Asp Gly Met Gly
     50                  55                   60

Asp Ser Glu Ile Thr Ala Ala Arg Asn Tyr Ala Glu Gly Ala Gly Gly
 65                  70                   75                   80
```

```
Phe Phe Lys Gly Ile Asp Ala Leu Pro Leu Thr Gly Gln Tyr Thr His
                85                  90                  95

Tyr Ala Leu Asn Lys Lys Thr Gly Lys Pro Asp Tyr Val Thr Asp Ser
            100              105                  110

Ala Ala Ser Ala Thr Ala Trp Ser Thr Gly Val Lys Thr Tyr Asn Gly
        115              120                  125

Ala Leu Gly Val Asp Ile His Glu Lys Asp His Pro Thr Ile Leu Glu
    130              135                  140

Met Ala Lys Ala Ala Gly Leu Ala Thr Gly Asn Val Ser Thr Ala Glu
145                  150                  155                  160
        153
Leu Gln His Ala Thr Pro Ala Ala Leu Val Ala His Val Thr Ser Arg
                165                  170                  175

Lys Cys Tyr Gly Pro Ser Ala Thr Ser Glu Lys Cys Pro Gly Asn Ala
            180                  185                  190

Leu Glu Lys Gly Gly Lys Gly Ser Ile Thr Glu Gln Leu Leu Asn Ala
        195                  200                  205

Arg Ala Asp Val Thr Leu Gly Gly Gly Ala Lys Thr Phe Ala Glu Thr
    210                  215                  220

Ala Thr Ala Gly Glu Trp Gln Gly Lys Thr Leu Arg Glu Gln Ala Gln
225                  230                  235                  240

Ala Arg Gly Tyr Gln Leu Val Ser Asp Ala Ala Ser Leu Asn Ser Val
            245                  250                  255

Thr Glu Ala Asn Gln Gln Lys Pro Leu Leu Gly Leu Phe Ala Asp Gly
            260                  265                  270

Asn Met Pro Val Arg Trp Leu Gly Pro Lys Ala Thr Tyr His Gly Asn
            275                  280                  285

Ile Asp Lys Pro Ala Val Thr Cys Thr Pro Asn Pro Gln Arg Asn Asp
    290                  295                  300

Ser Val Pro Thr Leu Ala Gln Met Thr Asp Lys Ala Ile Glu Leu Leu
305                  310                  315                  320

Ser Lys Asn Glu Lys Gly Phe Phe Leu Gln Val Glu Gly Ala Ser Ile
            325                  330                  335
    328      330
Asp His Gln Asn His Ala Ala Asn Pro Cys Gly Gln Ile Gly Glu Thr
        340                  345                  350

Val Asp Leu Asp Glu Ala Val Gln Arg Ala Leu Glu Phe Ala Lys Lys
        355                  360                  365

Glu Gly Asn Thr Leu Val Ile Val Thr Ala Asp His Ala His Ala Ser
    370                  375                  380

Gln Ile Val Ala Pro Asp Thr Lys Ala Pro Gly Leu Thr Gln Ala Leu
385                  390                  395                  400

Asn Thr Lys Asp Gly Ala Val Met Val Met Ser Tyr Gly Asn Ser Glu
            405                  410                  415
```

```
Glu Asp Ser Gln Glu His Thr Gly Ser Gln Leu Arg Ile Ala Ala Tyr
            420             425             430

Gly Pro His Ala Ala Asn Val Val Gly Leu Thr Asp Gln Thr Asp Leu
        435             440             445

Phe Tyr Thr Met Lys Ala Ala Leu Gly Leu Lys   *
    450             455             460
```

Dans cette séquence SEQ ID N°2, le fragment souligné correspond aux amino-acides ajoutés entre la proline et la valine, respectivement en positions 6 et 7, dans la phosphatase alcaline sauvage de *E.coli* ; les amino-acides en caractères gras correspondent aux amino-acides modifiés et les numéros en italique correspondent aux positions des aminoacides correspondants dans la phosphatase alcaline sauvage de *E. coli.*

Bien que la séquence ci-dessus corresponde au mutant D153H/K328H/D330N, l'invention comprend toutefois également les séquences correspondant aux mutants D330N, D153H/D330N et K328H/D330N, ainsi que les autres mutants précités.

De manière inattendue, des BAP modifiées comme précisé ci-dessus, présentent à la fois :

-   des propriétés catalytiques améliorées (notamment amélioration de l'affinité de l'enzyme pour le substrat), aptes à permettre une diminution du temps de révélation, lors de son utilisation dans un test de dosage jusqu'à un facteur 6 par rapport au réactif présentant la séquence bactérienne initiale et/ou d'augmenter la sensibilité de ce test et
-   une stabilité thermique de l'ordre de celle de la séquence bactérienne sauvage (BAP).

La présente invention a également pour objet des séquences nucléiques codant pour l'une quelconque des protéines telles que définies ci-dessus.

Parmi lesdites séquences, on peut citer :

-   les séquences modifiées, obtenues à partir de la séquence sauvage et
-   des séquences modifiées incluant 27 bases supplémentaires entre les bases 18 et 19, c'est-à-dire entre le codon codant pour la proline en position 6 de la phosphatase alcaline sauvage et le codon codant pour la valine en position 7, conformément à la SEQ ID N°1 :

```
GTGAAACAAA GCACTATTGC ACTGGCACTC TTACCGTTAC TGTTTACCCC TGTGACAAAA   60

GCC CGG ACA CCA GAA ATG CCC GTC GAC TTC AGT CGA CGA GCT CCC GGG   108
Arg Thr Pro Glu Met Pro Val Asp Phe Ser Arg Arg Ala Pro Gly
                  5               10                      15

GTT CTG GAA AAC CGG GCT GCT CAG GGC GAT ATT ACT GCA CCC GGC GGT   156
Val Leu Glu Asn Arg Ala Ala Gln Gly Asp Ile Thr Ala Pro Gly Gly
                20                  25                      30

GCT CGC CGT TTA ACG GGT GAT CAG ACT GCC GCT CTG CGT GAT TCT CTT   204
Ala Arg Arg Leu Thr Gly Asp Gln Thr Ala Ala Leu Arg Asp Ser Leu
                35                  40                  45

AGC GAT AAA CCT GCA AAA AAT ATT ATT TTG CTG ATT GGC GAT GGG ATG   252
Ser Asp Lys Pro Ala Lys Asn Ile Ile Leu Leu Ile Gly Asp Gly Met
            50                  55                  60

GGG GAC TCG GAA ATT ACT GCC GCA CGT AAT TAT GCC GAA GGT GCG GGC   300
Gly Asp Ser Glu Ile Thr Ala Ala Arg Asn Tyr Ala Glu Gly Ala Gly
            65                  70                  75

GGC TTT TTT AAA GGT ATA GAT GCC TTA CCG CTT ACC GGG CAA TAC ACT   348
Gly Phe Phe Lys Gly Ile Asp Ala Leu Pro Leu Thr Gly Gln Tyr Thr
80                  85                  90                  95

CAC TAT GCG CTG AAT AAA AAA ACC GGC AAA CCG GAC TAC GTC ACC GAC   396
His Tyr Ala Leu Asn Lys Lys Thr Gly Lys Pro Asp Tyr Val Thr Asp
                    100                 105                 110

TCG GCT GCA TCA GCA ACC GCC TGG TCA ACC GGT GTC AAA ACC TAT AAC   444
Ser Ala Ala Ser Ala Thr Ala Trp Ser Thr Gly Val Lys Thr Tyr Asn
                115                 120                 125

GGC GCG CTG GGC GTC GAT ATT CAC GAA AAA GAT CAC CCA ACG ATT CTG   492
Gly Ala Leu Gly Val Asp Ile His Glu Lys Asp His Pro Thr Ile Leu
            130                 135                 140

GAA ATG GCA AAA GCC GCA GGT CTG GCG ACC GGT AAC GTT TCT ACC GCA   540
Glu Met Ala Lys Ala Ala Gly Leu Ala Thr Gly Asn Val Ser Thr Ala
        145                 150                 155

                153
GAG TTG CAG CAC GCC ACG CCC GCT GCG CTG GTG GCA CAT GTG ACC TCG   588
Glu Leu Gln His Ala Thr Pro Ala Ala Leu Val Ala His Val Thr Ser
160                 165                 170                 175

CGC AAA TGC TAC GGT CCG AGC GCG ACC AGT GAA AAA TGT CCG GGT AAC   636
Arg Lys Cys Tyr Gly Pro Ser Ala Thr Ser Glu Lys Cys Pro Gly Asn
                    180                 185                 190

GCT CTG GAA AAA GGC GGA AAA GGA TCG ATT ACC GAA CAG CTG CTT AAC   684
Ala Leu Glu Lys Gly Gly Lys Gly Ser Ile Thr Glu Gln Leu Leu Asn
                195                 200                 205

GCT CGT GCC GAC GTT ACG CTT GGC GGC GGC GCA AAA ACC TTT GCT GAA   732
Ala Arg Ala Asp Val Thr Leu Gly Gly Gly Ala Lys Thr Phe Ala Glu
            210                 215                 220

ACG GCA ACC GCT GGT GAA TGG CAG GGA AAA ACG CTG CGT GAA CAG GCA   780
Thr Ala Thr Ala Gly Glu Trp Gln Gly Lys Thr Leu Arg Glu Gln Ala
        225                 230                 235

CAG GCG CGT GGT TAT CAG TTG GTG AGC GAT GCT GCC TCA CTG AAT TCG   828
Gln Ala Arg Gly Tyr Gln Leu Val Ser Asp Ala Ala Ser Leu Asn Ser
240                 245                 250                 255
```

```
GTG ACG GAA GCG AAT CAG CAA AAA CCC CTG CTT GGC CTG TTT GCT GAC    876
Val Thr Glu Ala Asn Gln Gln Lys Pro Leu Leu Gly Leu Phe Ala Asp
            260                 265                 270

GGC AAT ATG CCA GTG CGC TGG CTA GGA CCG AAA GCA ACG TAC CAT GGC    924
Gly Asn Met Pro Val Arg Trp Leu Gly Pro Lys Ala Thr Tyr His Gly
            275                 280                 285

AAT ATC GAT AAG CCC GCA GTC ACC TGT ACG CCA AAT CCG CAA CGT AAT    972
Asn Ile Asp Lys Pro Ala Val Thr Cys Thr Pro Asn Pro Gln Arg Asn
            290                 295                 300

GAC AGT GTA CCA ACC CTG GCG CAG ATG ACC GAC AAA GCC ATT GAA TTG   1020
Asp Ser Val Pro Thr Leu Ala Gln Met Thr Asp Lys Ala Ile Glu Leu
            305                 310                 315

TTG AGT AAA AAT GAG AAA GGC TTT TTC CTG CAA GTT GAA GGT GCG TCA   1068
Leu Ser Lys Asn Glu Lys Gly Phe Phe Leu Gln Val Glu Gly Ala Ser
320                 325                 330                 335
        328     330
ATC GAT CAC CAG AAT CAT GCT GCG AAT CCT TGT GGG CAA ATT GGC GAG   1116
Ile Asp His Gln Asn His Ala Ala Asn Pro Cys Gly Gln Ile Gly Glu
            340                 345                 350

ACG GTC GAT CTC GAT GAA GCC GTA CAA CGG GCG CTG GAA TTC GCT AAA   1164
Thr Val Asp Leu Asp Glu Ala Val Gln Arg Ala Leu Glu Phe Ala Lys
            355                 360                 365

AAG GAG GGT AAC ACG CTG GTC ATA GTC ACC GCT GAT CAC GCC CAC GCC   1212
Lys Glu Gly Asn Thr Leu Val Ile Val Thr Ala Asp His Ala His Ala
            370                 375                 380

AGC CAG ATT GTT GCG CCG GAT ACC AAA GCT CCG GGC CTC ACC CAG GCG   1260
Ser Gln Ile Val Ala Pro Asp Thr Lys Ala Pro Gly Leu Thr Gln Ala
            385                 390                 395

CTA AAT ACC AAA GAT GGC GCA GTG ATG GTG ATG AGT TAC GGG AAC TCC   1308
Leu Asn Thr Lys Asp Gly Ala Val Met Val Met Ser Tyr Gly Asn Ser
400                 405                 410                 415

GAA GAG GAT TCA CAA GAA CAT ACC GGC AGT CAG TTG CGT ATT GCG GCG   1356
Glu Glu Asp Ser Gln Glu His Thr Gly Ser Gln Leu Arg Ile Ala Ala
            420                 425                 430

TAT GGC CCG CAT GCC GCC AAT GTT GTT GGA CTG ACC GAC CAG ACC GAT   1404
Tyr Gly Pro His Ala Ala Asn Val Val Gly Leu Thr Asp Gln Thr Asp
            435                 440                 445

CTC TTC TAC ACC ATG AAA GCC GCT CTG GGG CTG AAA TAA               1443
Leu Phe Tyr Thr Met Lys Ala Ala Leu Gly Leu Lys
            450                 455
```

Dans cette séquence SEQ ID N°1, le fragment souligné correspond aux bases ajoutées entre le codon proline et le codon valine ; les séquences en caractères gras correspondent aux codons mutés et les numéros en italique correspondent aux positions des aminoacides correspondants dans la phosphatase alcaline sauvage de *E. coli.*

Bien que la séquence ci-dessus code pour le mutant D153H/K328H/D330N, l'invention comprend toutefois également les séquences codant pour le mutant D330N, le mutant D153H/D330N et le mutant K328H/D330N, ainsi que les autres mutants précités.

La présente invention a également pour objet un plasmide recombinant, caractérisé en ce qu'il comprend une séquence d'acide nucléique codant pour une phosphatase alcaline modifiée conformément à l'invention et apte à exprimer ladite protéine dans une cellule hôte convenable.

Conformément à l'invention, ledit plasmide comprend avantageusement des séquences régulant l'expression de la séquence d'acide nucléique codant pour la phosphatase alcaline modifiée.

On entend par séquences régulatrices, des séquences de type promoteur et terminateur actives ; on peut citer, à titre d'exemple, le promoteur du gène de la phosphatase alcaline (promoteur *phoA*) ou bien le promoteur *tac,* associé au répresseur *lacI^Q* (Carrier et al., J. Immunol. Methods, 1995, 177-186 et Szmelcman et al., J. acquired Immune Defic. Syndr., 1990, 3, 859).

La présente invention a également pour objet une cellule hôte, transformée par un plasmide selon l'invention, notamment une bactérie telle que *E. coli,* ladite cellule hôte étant déficiente pour le gène chromosomique de la phosphatase alcaline, ou n'exprimant pas le gène chromosomique de la phosphatase alcaline dans les conditions où le gène de la phosphatase alcaline porté par le plasmide selon l'invention s'exprime.

La présente invention a également pour objet un procédé de sélection d'une phosphatase alcaline bactérienne modifiée, possédant à la fois une activité catalytique améliorée par rapport à celle de la phosphatase alcaline bactérienne native et une stabilité thermique de l'ordre de celle de ladite phosphatase alcaline native, caractérisé en ce qu'il comprend :

i) la préparation d'une phosphatase alcaline chimère inactive comprenant l'introduction dans la séquence codant pour la BAP, d'au moins deux codons codant pour deux résidus d'amino-acides d'une phosphatase alcaline de mammifère (CIP, notamment) ;
ii) la réalisation d'une mutagénèse aléatoire ou dirigée sur le gène codant pour cette phosphatase alcaline chimère inactive ;
iii) l'expression des phosphatases alcalines obtenues en ii) ;
iv) la sélection des clones bactériens exprimant une phosphatase alcaline dont l'activité enzymatique est restaurée ; et
v) le séquençage des phosphatases alcalines modifiées ainsi obtenues et la sélection de la/les mutations de compatibilité à introduire dans une phosphatase alcaline bactérienne sauvage, pour construire ladite phosphatase alcaline bactérienne modifiée active, ayant les propriétés améliorées telles que précisées ci-dessus (activité catalytique augmentée par rapport à celle de la phosphatase alcaline bactérienne native et stabilité thermique de l'ordre de celle de ladite phosphatase alcaline native).

L'étape iv) permet d'obtenir des phosphatases alcalines dites révertantes, c'est-à-dire dont l'activité enzymatique est restaurée, par rapport au produit de l'étape i).

A titre d'exemple, le mutant obtenu à l'étape i) est par exemple le mutant D153H/K328H/Q329G/D330H, correspondant à une phosphatase alcaline chimère inactive et le mutant obtenu à l'étape iv) est par exemple le mutant D153H/K328H/Q329G/D330N, le mutant D153H/K328H/Q329A/D330H, le mutant D153H/K328H/Q329G/D330A ou le mutant D153H/K328H/Q329G/D330L, qui sont des enzymes révertantes, c'est-à-dire biologiquement actives.

Les mutations à l'origine de cette réversion phénotypique sont appelées mutations de compatibilité car elles permettent à deux séquences d'origines différentes de s'adapter l'une à l'autre pour former une protéine modifiée, biologiquement fonctionnelle.

L'étape v) permet la sélection desdites mutations de compatibilité, de manière à construire une phosphatase alcaline bactérienne modifiée active et stable thermiquement, présentant des propriétés enzymatiques (kcat et/ou $K_M$) améliorées par rapport à l'enzyme sauvage.

Conformément à ce procédé, ladite mutation de compatibilité [sélectionnée aux étapes iv) et v)] confère à une enzyme chimère phosphatase alcaline bactérienne/phosphatase alcaline de mammifère initialement inactive, des propriétés catalytiques performantes. Transférée dans la phosphatase alcaline bactérienne, cette mutation a pour effet d'améliorer les propriétés catalytiques de l'enzyme. Enfin, associée à l'une des autres mutations comme précisé ci-dessus et notamment à la mutation D153H, elle conduit à une enzyme dont les propriétés sont proches de celles des enzymes de mammifère, tout en ayant une stabilité thermique proche de celle de l'enzyme bactérienne.

Selon un mode de mise en oeuvre avantageux de l'invention, les mutations introduites à l'étape i) portent au moins sur les résidus d'amino-acide en positions 153, 328, 329 et 330 de l'enzyme bactérienne.

De préférence, elles portent sur l'ensemble des résidus suivants :

résidu 153 : remplacement de Asp par His,
résidu 328 : remplacement de Lys par His,
résidu 329 : remplacement de Gln par Gly,
résidu 330 : remplacement de Asp par His.

On obtient, en particulier, le mutant D153H/K328H/Q329G/D330H (phosphatase alcaline chimère inactive).

Selon un autre mode de mise en oeuvre dudit procédé, la mutation introduite à l'étape ii) porte au moins sur les résidus d'aminoacides en position 329 et/ou 330 de l'enzyme bactérienne et consiste notamment en la substitution de His en 330 par Asn, par Ala ou par Leu et/ou consiste en la substitution de Gly en position 329 par Ala ; conduisant à l'obtention des révertants biologiquement actifs précités, à savoir : D153H/K328H/Q329G/D330N, D153H/K328H/Q329G/ D330A, D153H/K328H/Q329G/D330L ou D153H/K328H/Q329A/D330H.

La présente invention a également pour objet un réactif de diagnostic, caractérisé en ce qu'il comprend une phosphatase alcaline modifiée selon l'invention.

Le réactif de diagnostic selon l'invention peut avantageusement être préparé par les voies classiques (couplage chimique) ou par génie génétique, notamment en utilisant une construction telle que décrite dans les Demandes de Brevet européen n°0 407 259 et n°0 556 111.

Les réactifs selon la présente invention trouvent notamment application dans des dosages immuno-enzymatiques ; leurs avantages par rapport aux réactifs incluant la séquence bactérienne sauvage de phosphatase alcaline, sont illustrés, par exemple, soit par une réduction du temps de révélation, jusqu'à un facteur 6 et/ou une augmentation de la sensibilité du test, ou par une augmentation du signal.

Outre les dispositions qui précèdent, l'invention comprend encore d'autres dispositions, qui ressortiront de la description qui va suivre, qui se réfère à des exemples de mise en oeuvre du procédé objet de la présente invention, ainsi qu'aux dessins annexés, dans lesquels :

- la figure 1 représente le plasmide de pLIP4.0.B apte à exprimer les gènes codant pour une phosphatase modifiée,
- la figure 2 représente la séquence nucléotidique du vecteur pLIP4.0.B, dans laquelle la séquence codante du gène phoA est en gras,
- la figure 3 illustre la stabilité thermique des différentes phosphatases alcalines produites,
- la figure 4 illustre la comparaison des activités enzymatiques des différents traceurs toxine-phosphatase fixés spécifiquement sur un anticorps anti-toxine adsorbé sur plaque de microtitration (mesure de la liaison spécifique obtenue en retranchant la liaison non spécifique de la liaison totale),
- les figures 5A et 5B illustrent l'effet des mutations sur le temps de révélation et la sensibilité d'un dosage immunoenzymatique par compétition de la toxine,
- la figure 6 représente la séquence du gène inséré codant pour la proinsuline et
- la figure 7A représente la comparaison des temps de révélation nécessaires au dosage de l'insuline en fonction du traceur utilisé et la figure 7B illustre l'effet des mutations sur la sensibilité d'un dosage immunoenzymatique par compétition de l'insuline.

Il doit être bien entendu, toutefois, que ces exemples sont donnés à titre d'illustration de l'objet de l'invention, dont ils ne constituent en aucune manière une limitation.

**EXEMPLE 1 : Construction de gènes chimères codant pour une phosphatase alcaline bactérienne modifiée.**

Ces constructions ont été réalisées par mutagénèse dirigée (KUNKEL et al., Methods Enzymol., 1987, **154**, 367-382), sur un dérivé du gène naturel de la phosphatase alcaline bactérienne.

Le vecteur initial est le plasmide pLIP4.0 (GILLET et al., Analytical Chemistry, 1993, **65**, 1779-1784), dérivé du plasmide pJC2431 possédant le gène sauvage de la phosphatase alcaline bactérienne (LAZZARONI et al., J. Bacteriol., 1985, **164**, 1376-1380). Il contient le gène de la phosphatase alcaline modifié dans sa partie 5' codante par insertion de plusieurs sites de restriction destinés à permettre l'intégration d'un gène étranger.

Par ailleurs, un site de restriction BamHI a été introduit par mutagénèse dirigée sur ce vecteur, en amont de la séquence de Shine-Dalgarno du gène phoA. L'oligonucléotide utilisé pour cette expérience correspond à la SEQ ID N°3 :

**TGTACAAATACATTAAAGGATCCAAACAAAGCGACTAT**

(le site BamHI est indiqué en gras dans la séquence).

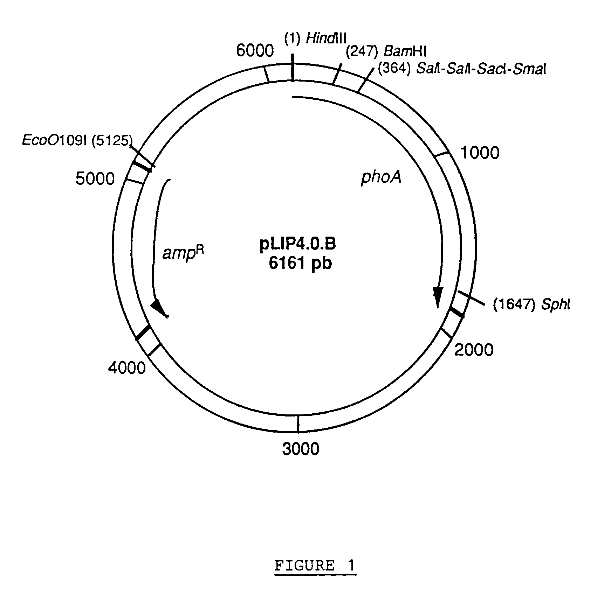

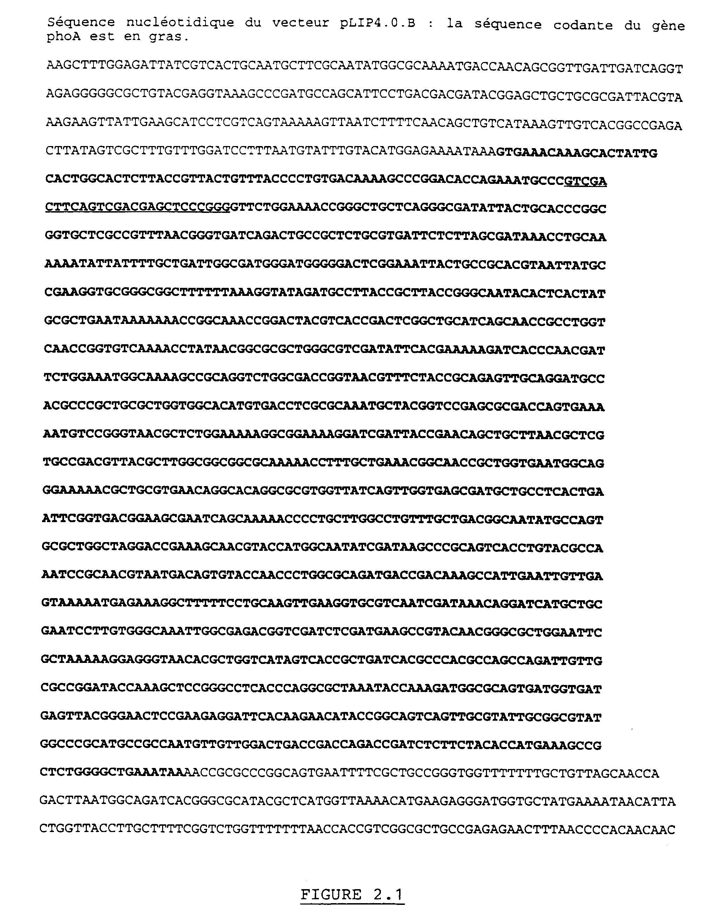

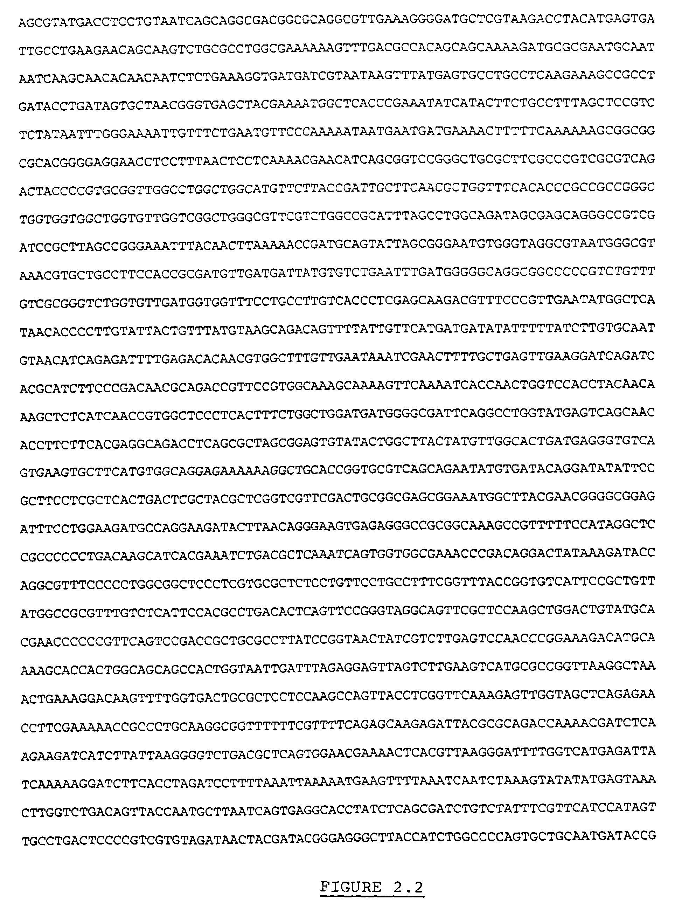

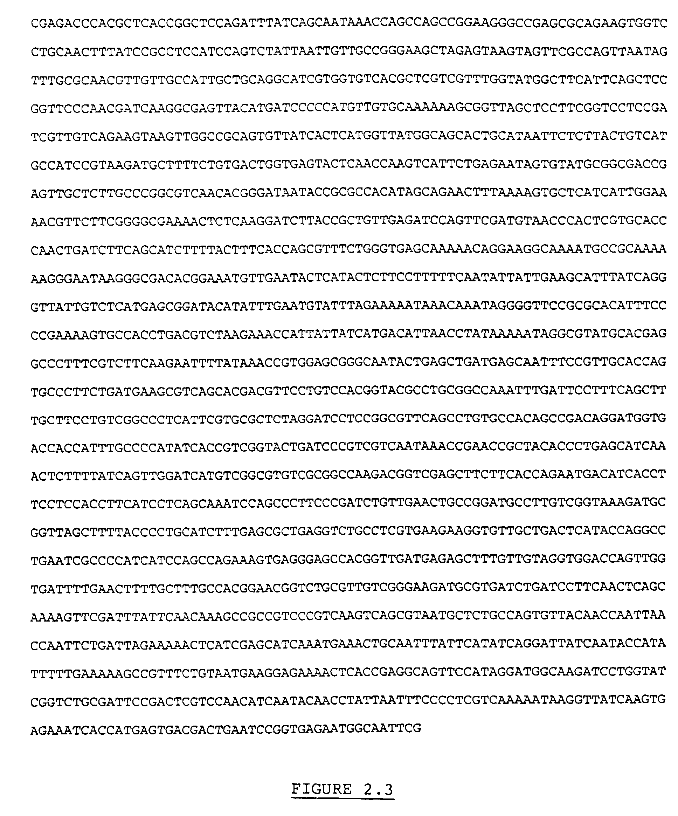

Le promoteur phoA, encadré par les sites Eco0109 I ou HindIII et BamHI, peut ainsi être changé à volonté. Le vecteur résultant est nommé pLIP4.0.B (figure 1 : représentation schématique du vecteur pLIP4.0.B et figure 2 : séquence nucléotidique du vecteur pLIP4.0.B).

Trois mutations ont été introduites simultanément dans le gène de la phosphatase alcaline d'*E. coli,* afin de substituer les résidus 328, 329 et 330 de l'enzyme bactérienne par les résidus correspondants présents dans les phosphatases de mammifère. Cette expérience a été réalisée sur un fragment SacI/SphI du gène de la phosphatase (phoA) inséré dans un phage M13 avec l'oligonucléotide de synthèse correspondant à la SEQ ID N°4 ;

**GATTCGCAGCATGATGACCGTGATCGATTGACGC**

(la séquence modifiée est indiquée en gras).

Le fragment muté a ensuite été réinséré dans le vecteur pLIP4.0.B et la séquence nucléique contrôlée à nouveau. La mutation D153H a été indépendamment construite selon un protocole identique, avec un oligonucléotide adapté, et ajoutée aux trois mutations précédentes par recombinaison génétique. Le quadruple mutant obtenu est le suivant : D153H/K328H/Q329G/D330H. La première lettre indique le résidu initialement présent sur la phosphatase alcaline bactérienne, le nombre correspond à la position du résidu dans la séquence bactérienne sauvage et la seconde lettre représente la mutation introduite qui correspond à l'acide aminé présent dans les phosphatases de mammifères.

**EXEMPLE 2 : Production, purification et caractéristiques enzymatiques des phosphatases alcalines chimères.**

Les différentes constructions génétiques décrites dans l'exemple 1, ainsi que le vecteur pJC2431 contenant le gène sauvage de la phosphatase alcaline bactérienne, ont été introduits dans la souche de *E. coli* CC118 déficiente pour le gène chromosomique de la phosphatase alcaline (MANOIL et BECKWITH, P.N.A.S., 1985, **82**, 8129-8131).

Les clones bactériens ont été cultivés sur un milieu contenant le substrat 5-bromo-4-chloro-3-indolyl phosphate. Les colonies exprimant une phosphatase alcaline possédant une activité spécifique suffisamment élevée hydrolysent le substrat et apparaissent en bleu. C'est le cas des bactéries contenant les plasmides pJC2431 et pLIP4.0.B. Le clone ayant intégré le plasmide pLIP4.0.B.-D153H/K328H/Q329G/D330H par contre est blanc et apparaît incapable d'hydrolyser le substrat colorimétrique. La protéine produite est donc dépourvue d'activité enzymatique.

La purification des différentes phosphatases alcalines est effectuée à partir de 200 ml de culture bactérienne. Les protéines périplasmiques sont extraites par choc osmotique (NEU et HEPPEL, J. Biol. Chem., 1965, **240**, 3685-3692), puis concentrées 20 fois et dialysées contre un tampon Tris-HCl 20 mM pH 8, MgCl$_2$ 1 mM sur membrane Centricon® 30. Les protéines sont séparées par isoélectrofocalisation sur une colonne Mono P HR® 5/5 Pharmacia.

Les différentes phosphatases alcalines sont ensuite isolées des composants du tampon sur un tamis moléculaire (G-75 Pharmacia) et conservées dans un milieu contenant 10 mM de MgCl$_2$. La pureté des protéines obtenues est contrôlée sur un gel polyacrylamide-SDS.

La mobilité électrophorétique observée est identique pour toutes les protéines produites à partir des vecteurs dérivés de pLIP4. Elle est inférieure à celle obtenue pour une phosphatase alcaline commerciale (Sigma) ou produite à partir du gène sauvage contenu dans le vecteur pJC2431. Cette différence correspond à 1000 da environ. Elle est en accord avec la présence des 9 acides aminés supplémentaires présents dans la partie N-terminale des phosphatases spécifiés par les vecteurs dérivés de pLIP4. Ces résidus correspondent aux sites de restriction additionnels introduits sur le gène des vecteurs pLIP4. Une mesure au scanner montre que cette bande représente plus de 95 % des protéines colorées sur le gel.

Les constantes cinétiques (kcat et Km) des enzymes ont été mesurées en utilisant le para-nitrophényl-phosphate (pNPP) comme substrat, à 25°C dans un tampon Tris-HCl 1M pH 8,0 et en mesurant la libération de p-nitrophénolate à 410 nm, comme illustré au Tableau I ci-après, dans les conditions suivantes : préincubation en Tris-HCl 1 M pH 8,0 Mg$^{2+}$ 10 mM, réaction en Tris-HCl 1 M pH 8,0.

## TABLEAU I

| | kcat (s$^{-1}$) | K$_M$ (µM) | kcat/K$_M$ (10$^6$ M$^{-1}$.s$^{-1}$) |
|---|---|---|---|
| pJC2431 | 65 ± 1 | 23 ± 0,1 | 2,8 |
| pLIP4.0.B | 78 ± 4 | 30 ± 5 | 2,6 |
| D153H/K328H/Q329G/D330H | < 0,7 | ND | ND |

Les valeurs de kcat et Km ont été obtenues à partir de la représentation graphique de Eadie et Hofstee (vi=f(vi/ [s])) (L. PENASSE, Les enzymes : cinétique et mécanismes d'action, MASSON et CIE, eds, 1974) et calculées à l'aide du logiciel KALEIDAGRAPH® (publié par Synergy Software). L'enzyme obtenue à partir du plasmide pLIP4.0.B présente des paramètres cinétiques voisins de ceux de l'enzyme de type sauvage. La présence d'une insertion dans la région N-terminale de la protéine ne modifie donc pas de façon significative l'activité catalytique de l'enzyme.

L'addition de la mutation D153H à ces modifications rend l'enzyme totalement silencieuse alors qu'elle continue à être produite normalement par la bactérie. Ainsi, l'introduction de 4 résidus issus de mammifère dans la phosphatase alcaline bactérienne est incompatible avec le maintien d'une forme enzymatiquement fonctionnelle. La présence de deux d'entre eux seulement, les mutations D153H et K328H a un effet limité sur les propriétés enzymatiques (JANEWAY et al., Biochemistry, 1993, **32**, 1601-1609). La boucle 328-330 et plus précisément les résidus 329 et 330 jouent donc un rôle important dans la perte d'activité observée.

**EXEMPLE 3 : Mutagénèse aléatoire sur la chimère inactive, sélection de révertants phénotypiques, identification des mutations à l'origine de cette réversion.**

Une mutagénèse aléatoire est réalisée sur la portion SacI-SphI du quadruple mutant de phosphatase alcaline par la technique de PCR (*Polymerase Chain Reaction*) (SAIKI et al., Science, 1988, **239**, 487-491).

L'expérience est réalisée avec la Taq polymérase (BRL), qui commet en moyenne une erreur pour 30 000 nucléotides incorporés, et les oligonucléotides correspondant respectivement aux SEQ ID N° 5 et N° 6 :

AACAACATTGGCG**GCATGC**GGGCC (le site SphI est en gras dans la séquence)

GACTTCA**GTCGAC**GAGCTCCCGGG (le site SacI est en gras dans la séquence).

Le nombre de cycles d'amplification est de 30. Le fragment de gène obtenu est contrôlé sur gel d'agarose et purifié sur Sephaglass® (Pharmacia). Il est ensuite digéré par les enzymes SacI et SphI puis réinséré dans le plasmide d'origine en place du fragment SacI-SphI non mutagénéisé.

La transformation des bactéries CC118 par le produit de ligation est effectuée par électroporation (Système Biorad) et les clones sont étalés sur milieu inducteur de la phosphatase alcaline contenant de l'ampicilline et un substrat colorimétrique de l'enzyme, le 5-bromo-4-chloro-3-indolyl phosphate, dont l'hydrolyse conduit à un produit de couleur bleue.

Deux clones bleus ont été obtenus pour 50 000 blancs. L'origine de cette réversion phénotypique a été recherchée par le séquençage des gènes correspondants. Il révèle la présence d'une seule et même mutation H330N chez ces deux clones. Ce type de mutation a été nommé mutation de compatibilité de par sa propriété à rendre compatible la présence sur un même gène de résidus appartenant à deux phosphatases d'origine différente et qui donne initialement une enzyme dépourvue d'activité catalytique.

**EXEMPLE 4 : Propriétés enzymatiques de la phosphatase alcaline produite par le clone révertant (vecteur pLIP4.0.B.-D153H/K328H/Q329G/H330N).**

La phosphatase alcaline synthétisée par le clone sélectionné dans l'exemple 3 a été purifiée selon la procédure décrite dans l'exemple 2 et ses propriétés catalytiques ont été mesurées à pH 8, comme illustré au Tableau II ci-après, dans les conditions suivantes : préincubation en Tris-HCl 1 M pH 8,0 Mg$^{2+}$ 10 mM, réaction en Tris-HCl 1 M pH 8,0.

## TABLEAU II

| | $k_{cat}$ (s$^{-1}$) | $K_M$ ($\mu$M) | $k_{cat}/K_M$ ($10^6$ M$^{-1}$.s$^{-1}$) |
|---|---|---|---|
| pLIP4.0.B | 78 ± 4 | 30 ± 5 | 2,6 |
| D153H/K328H/Q329G/H330N (révertant) | 82 ± 2 | 49 ± 6 | 1,7 |
| D330N | 148 ± 3 | 20 ± 2 | 7,2 |
| K328H | 78 ± 2 | 58 ± 2 | 1,3 |
| K328H/D330N | 53 ± 2 | 114 ± 9 | 0,5 |
| D153H | 110 ± 4 | 50 ± 5 | 2,2 |
| D153H/D330N | 215 ± 18 | 34 ± 7 | 6,4 |
| D153H/K328H/D330N | 160 ± 4 | 52 ± 6 | 3,1 |

L'activité catalytique mesurée est similaire à celle de l'enzyme bactérienne de type sauvage. L'affinité pour le substrat est légèrement diminuée.

La mutation de compatibilité H330N (qui ne correspond ni à un résidu naturel de l'enzyme, ni à un résidu présent sur les enzymes de mammifère) est donc capable de conférer des propriétés catalytiques performantes à l'enzyme mutée.

On a contrôlé, par ailleurs, que la présence d'un résidu 330D (résidu présent sur la phosphatase bactérienne) ne conduit pas à un tel résultat.

### EXEMPLE 5 : Propriétés de la mutation 330N seule ou en combinaison avec les différentes mutations de type mammifère.

L'impact de la mutation 330N dans un contexte phosphatase alcaline bactérienne sauvage ou en combinaison avec les mutations D153H et/ou K328H, a été évalué.

Pour réaliser cette étude, la mutation D330N a été introduite par mutagénèse dirigée dans le gène porté par le vecteur pLIP4.0.B. initial ou modifié par D153H, K328H ou D153H/K328H, par mutagénèse dirigée également.

Les protéines ont été produites et purifiées comme dans l'exemple 2.

Les paramètres cinétiques à pH 8 et à pH 10 ont été déterminés et sont indiqués dans le Tableau II ci-dessus et le Tableau III ci-après.

Par rapport aux constantes déterminées pour l'enzyme produite à partir du vecteur pLIP4.0.B., la mutation D330N induit à pH 8, une augmentation d'un facteur 2 de la vitesse de catalyse de l'enzyme modifiée associée à une augmentation de 30 % de l'affinité pour le substrat. En combinaison avec la mutation K328H, la vitesse de catalyse tombe sous le niveau de celle de l'enzyme initiale et l'affinité pour le substrat chute d'un facteur 3 à 4. La mutation K328H seule ne modifie pas la vitesse de catalyse de l'enzyme, mais provoque une chute d'affinité d'un facteur 2. Ce dernier résultat est en accord avec les travaux publiés par JANEWAY et al., Biochemistry, 1993, **32**, 1601-1609.

De manière inattendue, associée à la mutation D153H, la mutation D330N induit une augmentation de vitesse de catalyse d'un facteur proche de 3 et ne modifie pas de façon significative l'affinité pour le substrat.

Dans des conditions similaires, la mutation D153H seule augmente seulement 1,4 fois la vitesse de catalyse et diminue d'un facteur proche de 2 l'affinité pour le substrat.

Enfin, le triple mutant D153H/K328H/D330N voit sa vitesse de catalyse retomber au niveau de celle du mutant D330N seul et induit une baisse d'un facteur 2 de l'affinité pour le substrat.

Ce résultat est à comparer à ceux obtenus avec le double mutant D153H/K328H (JANEWAY et al., Biochemistry, 1993, **32**, 1601-1609) qui montrent une diminution d'un facteur 4 de la vitesse de catalyse et 3 de l'affinité pour le substrat par rapport à l'enzyme sauvage.

A pH 10, l'enzyme produit à partir du plasmide pLIP4.0.B présente une vitesse de catalyse similaire à celle observée en Tris 1 M à pH 8 et une affinité pour le substrat diminuée d'un facteur 3, comme illustré au Tableau III ci-après, dans les conditions suivantes: préincubation en CAPS (cyclohexylaminopropane sulfonic acid) 0,1 M NaCl 0,4 M pH 10,0 $Mg^{2+}$ 10 mM, réaction en CAPS 0,1 M NaCl 0,4 M pH 10,0 $Mg^{2+}$ 10 mM.

## TABLEAU III

| | kcat ($s^{-1}$) | $K_M$ ($\mu M$) | kcat/$K_M$ ($10^6$ $M^{-1}.s^{-1}$) |
|---|---|---|---|
| pLIP4.0.B | $80 \pm 3$ | $90 \pm 8$ | 0,9 |
| D153H/K328H/Q329G/H330N (révertant) | $227 \pm 4$ | $562 \pm 45$ | 0,4 |
| D330N | $201 \pm 10$ | $47 \pm 4$ | 4,3 |
| K328H | $214 \pm 11$ | $105 \pm 11$ | 2,0 |
| K328H/D330N | $254 \pm 20$ | $112 \pm 9$ | 2,3 |
| D153H | $240 \pm 12$ | $320 \pm 30$ | 0,8 |
| D153H/D330N | $1389 \pm 110$ | $350 \pm 30$ | 4,0 |
| D153H/K328H/D330N | $650 \pm 43$ | $170 \pm 16$ | 3,8 |

Ce résultat est voisin de celui observé par JANEWAY et al., 1993, sur l'enzyme sauvage. Là encore, l'insertion de plusieurs acides aminés en N-terminal de l'enzyme ne modifie pas son comportement de façon significative.

Le clone révertant possède une activité catalytique augmentée d'un facteur 3, mais une affinité pour le substrat diminuée d'un facteur 6. Le mutant D330N possède une vitesse de catalyse 2,5 fois plus élevée que l'enzyme non mutée, dans les mêmes conditions et une affinité qui s'élève d'un facteur 2.

Le double mutant K328H/D330N présente une activité 3 fois supérieure et une affinité diminuée légèrement de 20% environ. Le double mutant D153H/D330N présente une vitesse de catalyse augmentée 17 fois par rapport à l'enzyme initiale, 7 fois par rapport au simple mutant D330N et 6 fois par rapport au simple mutant D153H. Dans ce cas, il y a un véritable effet synergique entre ces deux mutations D153H/D330N. Par contre, l'affinité pour le substrat diminue d'un facteur 4 environ par rapport à l'enzyme bactérienne et cette chute peut être attribuée à la mutation D153H qui, seule, induit un effet similaire. L'association des trois mutations D153H/K328H/D330N engendre une vitesse de catalyse et une affinité intermédiaires par rapport aux valeurs obtenues pour les deux doubles mutants.

La combinaison D153H/D330N constitue la construction la plus efficace en terme de vitesse de catalyse qui atteint une valeur approchant celle de la CIP dont le kcat est de l'ordre de 2000 s$^{-1}$.

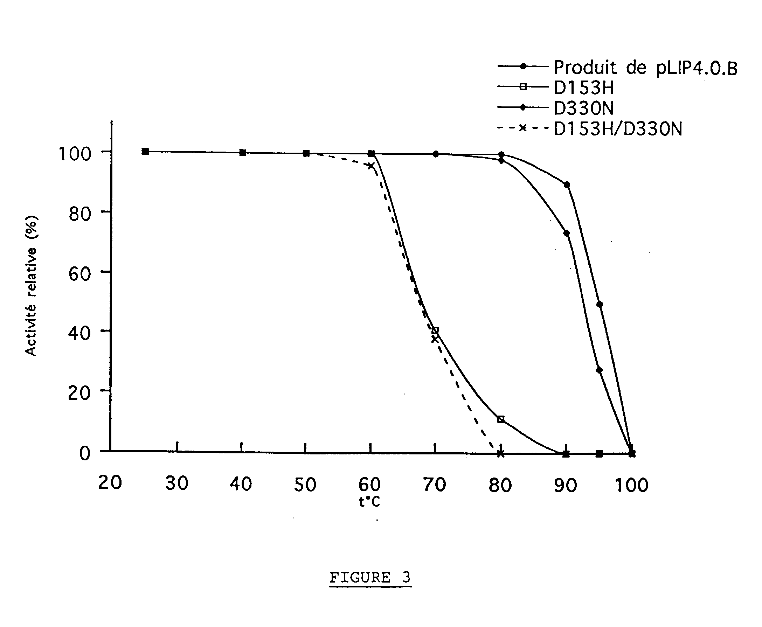

La stabilité thermique des enzymes produites par le vecteur pLIP4.0.B et les dérivés D153H, D330N et D153H/D330N a été mesurée (figure 3). Les enzymes sont préincubées 2 h à 25°C dans un tampon Tris 1 M pH 8 Mg$^{++}$ 100 mM.

Un aliquot est porté à la température indiquée pendant 15 min dans un tampon Tris 1 M pH 8, 10 mM Mg$^{++}$. Après refroidissement dans la glace, la mesure de l'activité enzymatique est réalisée en présence de 5 mM de paranitrophényl phosphate pendant 15 min à 25°C.

La protéine produite par le vecteur pLIP4.0.B (courbe -●-) et le mutant D330N (courbe -◇-) ont une température de demi-dénaturation de 95°C environ, similaire à celle de la phosphatase alcaline bactérienne naturelle. Ni l'insertion en N-terminal de plusieurs acides aminés, ni la mutation D330N ne provoquent une augmentation de sensibilité de l'enzyme à la température. Par contre, le mutant D153H (courbe -□-) et le double mutant D153H/D330N (-x-) ont une température de demi-dénaturation de 70°C environ, similaire à celle publiée pour le mutant D153H par JANEWAY et al., 1993. Cette valeur est nettement supérieure à la valeur publiée pour la CIP et qui se situe aux environs de 55°C ou 65°C en présence de Mg$^{2+}$ (conditions contrôles établies pour l'exemple) .

**EXEMPLE 6 : Construction de vecteurs de fusion toxine-mutants de phosphatase alcaline.**

Les vecteurs pLIP4.0.B, pLIP4,0.B/D330N, pLIP4.0.B./D153H/D330N ont été digérés par l'enzyme SalI et refermés sur eux-mêmes de façon à créer un déphasage du cadre de lecture du gène phoA. Les bactéries CC118 transformées par ces vecteurs apparaissent blanches sur milieu inducteur de PhoA contenant de l'ampicilline et le substrat 5-bromo-4-chloro-3-indolyl phosphate. Ces vecteurs déphasés sont nommés pLIP4.B, pLIP4.B/D330N, pLIP4.B/D153H/D330N.

Une réaction de PCR est réalisée sur la portion du vecteur pLIP1 (GILLET et al., Protein Ingeneering, 1992, **5**, 3, 273-278) portant le gène codant pour la toxine de venin de serpent érabutoxine a, de façon à introduire aux extrémités du gène les sites SalI et XmaI nécessaires au clonage dans les vecteurs pLIP4. Les oligonucléotides utilisés correspondent respectivement aux SEQ ID N° 7 et SEQ ID N° 8 :

GAAATGCCC**GTCGAC**AGGATATGTTTTAAC (le site SalI est en gras dans la séquence),

GAAC**CCCGGG**AGCTCCATTGTTGCAGACCT (le site XmaI est en gras dans la séquence).

Le nombre de cycles d'amplification est de 30. Le gène obtenu est contrôlé sur gel d'agarose « Low Melting Point » et extrait du gel par des traitements au phénol et au chloroforme. Il est ensuite digéré par les enzymes de restriction SalI et XmaI et inséré dans l'ADN double brin du phage M13mp18 afin d'en contrôler la séquence.

Puis le gène est inséré entre les sites SalI et XmaI des vecteurs pLIP4.B, pLIP4.B D330N, pLIP4.B D153H/D330N. Les bactéries CC118 sont transformées par le produit de ligation et étalées sur un milieu inducteur de la phosphatase alcaline contenant de l'ampicilline et le substrat 5-bromo-4-chloro-3-indolyl phosphate.

Les colonies exprimant la protéine de fusion érabutoxine a/Phosphatase alcaline (Ea/PhoA) hydrolysent le substrat et apparaissent en bleu. 21 clones bleus pour 300 clones blancs ont été obtenus pour la construction pLIP4.B/Ea, 4 clones bleus pour 100 blancs pour la construction pLIP4.B-D330N/Ea et 4 clones bleus pour 113 blancs pour la construction pLIP4.B-D153H-D330N/Ea. La présence de l'insert dans les constructions a été vérifiée par restriction SacI/XmaI (insert d'environ 200 pb) et la séquence du gène codant pour Ea a été de nouveau vérifiée par séquençage.

**EXEMPLE 7 : Production/extraction des traceurs immuno-enzymatiques.**

La production des différentes protéines de fusion est effectuée à partir de 400 ml de culture bactérienne. Les protéines périplasmiques sont extraites par choc osmotique puis concentrées sur membrane YM-30 AMICON jusqu'à un volume de 1 ml et dialysés contre un tampon Tris-HC 20 mM pH 8, $MgCl_2$ 10 mM, pour l'hybride non modifié et le mutant D330N; un tampon Tris-HC 20 mM pH 8, $MgCl_2$ 100 mM pour le mutant D153H/D330N. Les solutions sont conservées à -20°C en présence de $NaN_3$ 0,02 %.

**EXEMPLE 8 : Comparaison des activités enzymatiques des différents traceurs érabutoxine-a/phosphatase alcaline modifiée, fixés spécifiquement sur un anticorps anti-toxine adsorbé sur plaque de microtitration et effet des mutations sur le temps de révélation et la sensibilité d'un dosage immunoenzymatique par compétition de la toxine.**

1) <u>Activité enzymatique</u> :

L'anticorps monoclonal $M\alpha2$-3 spécifique des toxines (TREMEAU et al., FEBS Lett., 1986, **208**, 236-240) est adsorbé une nuit à 4°C sur plaque de microtitration à raison de 10 ng par puits dans un volume de 50 µl de tampon Tris-HCl 50 mM pH 7,4. Les puits sont ensuite saturés une nuit à 4°C avec 250 µl d'une solution Tris-HCl 100 mM pH 7,4 contenant 0,3 % de sérum albumine bovine.

5 lavages sont réalisés avec un tampon Tris-HCl 10 mM pH 7,4 0,05 % Tween® 2.

Les dilutions des différents traceurs sont réalisées dans un tampon Tris-HCl pH 7,4 100 mM, 0,1 % sérum albumine bovine, 10 mM $MgCl_2$. 50 µl de ces dilutions sont ajoutés dans les puits de titration et incubés une nuit à 4°C.

5 lavages sont réalisés avec un tampon Tris-HCl 10 mM pH 7,4 0,05 % Tween® 2.

La quantité de traceur fixée sur les plaques est révélée par addition de 200 µl de Tris-HCl 1 M pH 8, 10 mM $MgCl_2$, 10 mM para-nitro-phényl-phosphate (pNPP) pour le traceur non modifié et celui portant la mutation D330N; 200 µl de tampon CAPS 100 mM pH 10,0, 400 mM NaCl, 10 mM $MgCl_2$, 10 mM pNPP sont utilisés pour le traceur portant les deux mutations D153H/D330N. La densité optique est mesurée à 410 nm après 2 heures 30 et 24 h d'incubation à température ambiante.

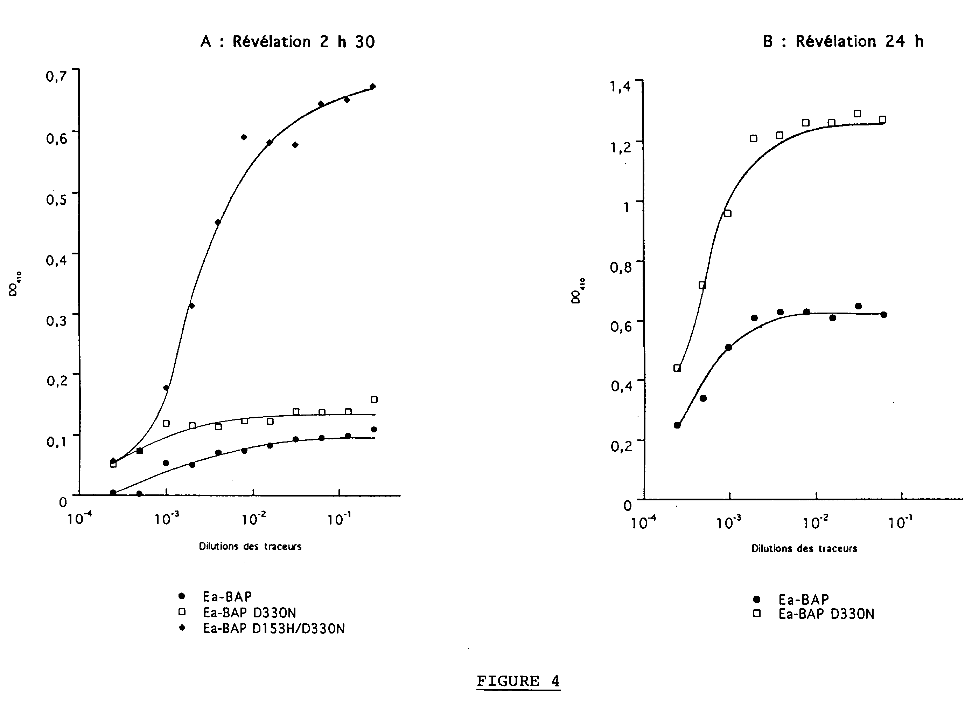

Les mêmes expériences sont réalisées en présence d'un excès de toxine (50 µl d'une solution de toxine à 1 mg/ml) afin de mesurer la liaison non spécifique qui est retranchée de la liaison totale. Les résultats sont reportés sur la figure 4.

2) <u>Effet des mutations sur le temps de révélation et la sensibilité</u> :

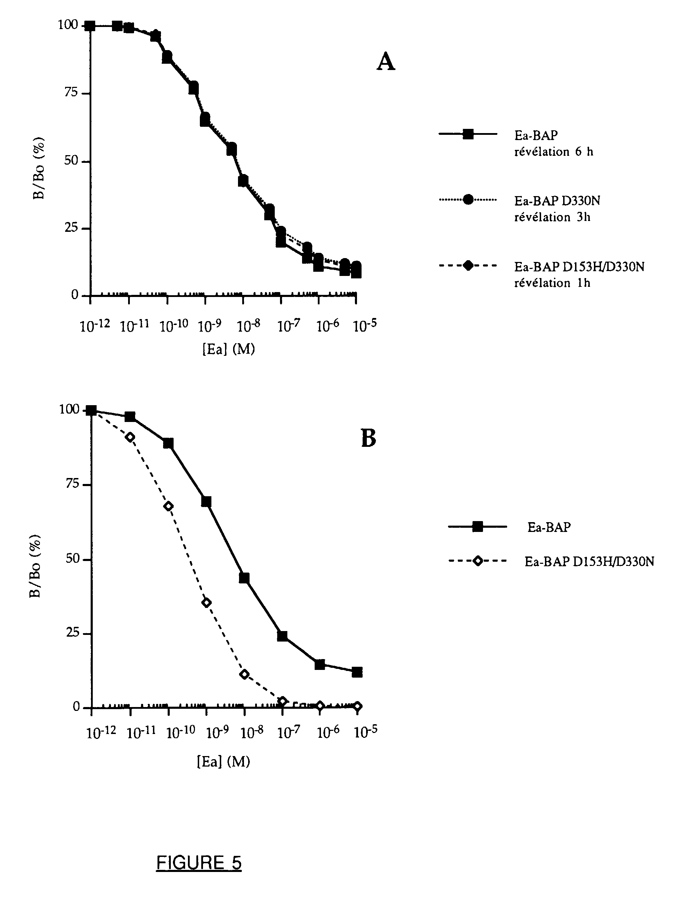

Des courbes d'étalonnage sont réalisées avec les plaques de microtitration préparées comme en 1), avec l'anticorps $M\alpha2$-3. Les différents traceurs sont utilisés à la même concentration et l'érabutoxine est utilisée en concentrations variables ($10^{-5}$ à $10^{-12}$ M) comme compétiteur. 200 µl de solution contenant 10 mM de substrat pNPP sont ajoutés dans un tampon Tris-HCl 1 M pH 8, 10 mM $MgCl_2$ pour le traceur non muté et pour le traceur muté en position D330N. L'incubation est réalisée dans un tampon CAPS 100 mM pH 10, 400 mM NaCl, 10 mM $MgCl_2$ pour le double mutant D153H/D330N. Les courbes de compétition standard obtenues sont représentées sur la figure 5A.

Dans une seconde série d'expériences réalisée dans les mêmes tampons que précédemment, la concentration du traceur contenant la double mutation D153H/D330N est diminuée d'un facteur 16, de façon à donner un même signal (0,5 DO à 410 nm en 6 heures) que le traceur ne contenant pas de mutation, en l'absence de compétiteur. Dans ces conditions, la quantité de toxine nécessaire pour inhiber 50 % du signal enzymatique est diminuée d'un facteur 17 lorsque l'on utilise le traceur portant les mutations (0,4 nM au lieu de 7 nM). Les résultats sont présentés à la figure 5B.

Ces résultats montrent que le temps de révélation du dosage est réduit d'un facteur 2 pour le mutant D330N et d'un facteur 6 pour le mutant D153H/D330N et que la sensibilité du dosage (exprimée pour la concentration d'antigène inhibant 50 % du signal) est améliorée d'un facteur 17 (cas du double mutant).

**EXEMPLE 9 : Construction des vecteurs de fusion proinsuline-mutants de phosphatase alcaline et production des traceurs immuno-enzymatiques.**

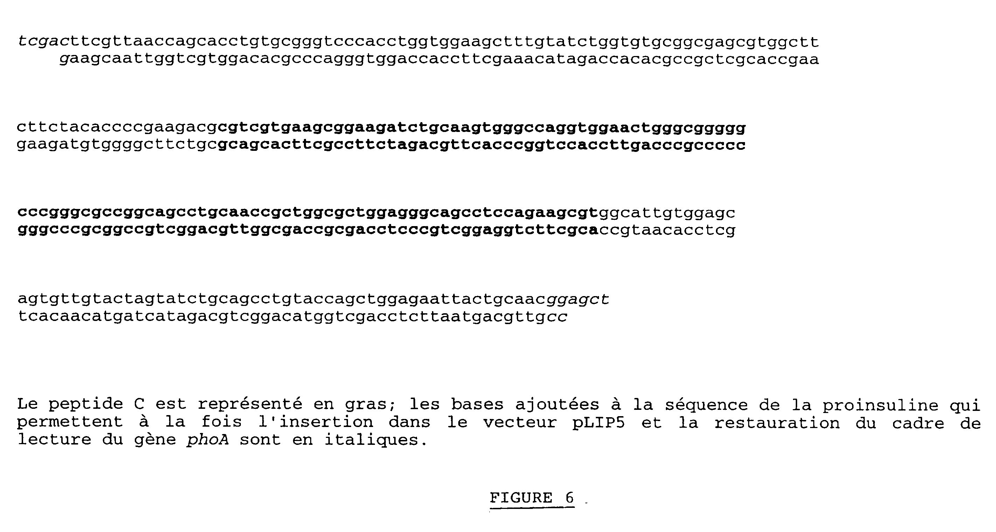

Un gène de synthèse codant pour la proinsuline humaine a été construit et inséré aux sites Sall et Sacl du vecteur pLIP5 (CARRIER et al., J.I.M., 1995, **181**, 177-186) qui est un dérivé du vecteur pLIP4.B dans lequel le promoteur phoA a été remplacé par le promoteur *tac* associé au répresseur *lacl*$^Q$. La séquence du gène inséré codant pour la proinsuline est représentée à la figure 6.

Les mutations D330N et D153H/D330N ont été introduites dans ce vecteur par recombinaison génétique entre les vecteurs pLIP4.0.B. portant ces mutations et le vecteur pLIP5-proinsuline.

Les constructions obtenues ont été vérifiées par séquençage. Les plasmides obtenus ont été utilisés pour transformer la souche *E. coli* W3110 (American Type Culture Collection n° 27325).

La production des différents traceurs proinsuline-phosphatase alcaline est effectuée à partir de 400 ml de culture bactérienne. Les échantillons sont traités comme dans l'exemple 7.

**EXEMPLE 10 : Effet des mutations sur le temps de révélation et la sensibilité d'un dosage immunoenzymatique par compétition de l'insuline.**

1) Comparaison des temps de révélation :

Dans un premier temps, la quantité des différents traceurs a été mesurée en équivalent insuline par un test commercial RIA par compétition (Kit INSI-PR), de façon à standardiser les solutions de traceurs utilisées dans les tests ELISA qui suivent. La même quantité de traceur sera ainsi introduite dans les différents tests réalisés.

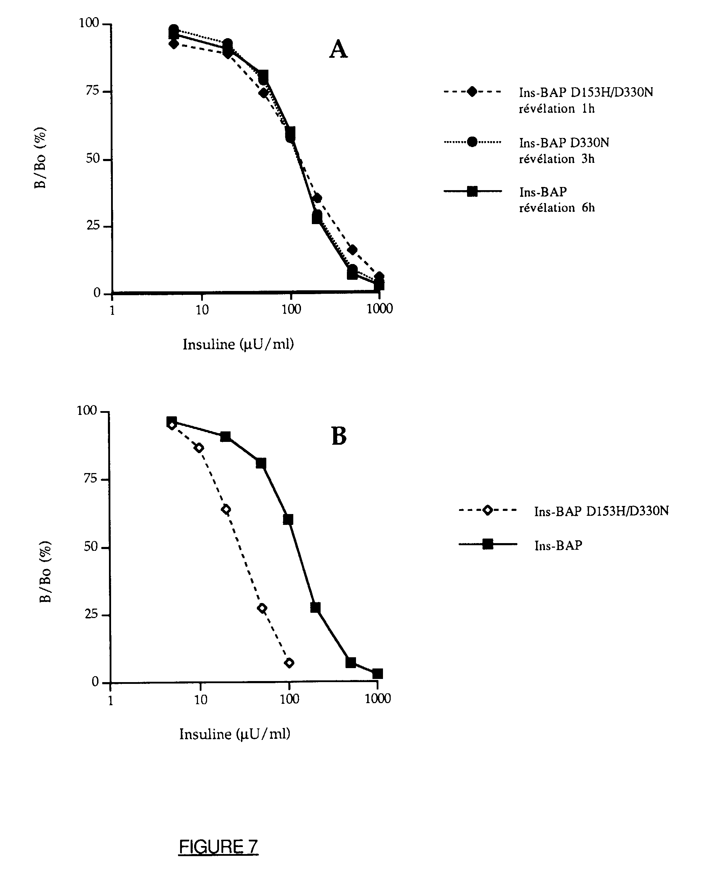

Des anticorps de chèvre anti-immunoglobuline de souris (IgG+IgM, H+L, Jackson ImmunoResearch Laboratories, Baltimore) ont été adsorbés sur des plaques de microtitration une nuit à 4°C dans un tampon Tris-HCl 50 mM pH 7,4 à raison de 100 µl par puits à la concentration de 10 µg/ml. Les puits sont saturés ensuite une nuit à 4°C avec 200 µl d'une solution de Tris-HCl 100 mM pH 7,4, 0,3 % sérum albumine bovine. Après lavages comme dans l'exemple 8, 50 µl d'une solution standardisée de traceur immunoenzymatique, 50 µl de différentes concentrations d'insuline humaine puis 50 µl d'une solution d'anticorps monoclonal anti-insuline, sont ajoutés dans les puits de microtitration et incubés une nuit à 4°C. Le tampon d'incubation est Tris-HCl 100 mM pH 7,4, 0,1 % sérum albumine bovine, 10 mM $MgCl_2$. Les puits sont lavés, puis 200 µl de tampon contenant 10 mM de substrat pNPP sont ajoutés dans un tampon Tris-HCl 1 M pH 8, 10 mM $MgCl_2$ pour le traceur non muté et pour le traceur muté en position D330N, alors que pour le traceur D153H/D330N, l'incubation est réalisée dans un tampon CAPS 100 mM pH 10, 400 mM NaCl, 10 mM $MgCl_2$. La lecture de la densité optique à 410 nm est réalisée lorsqu'elle atteint 0,5 en absence d'insuline. Les résultats sont présentés sur la figure 7A.

2) Effet des mutations sur le temps de révélation et la sensibilité d'un dosage immunoenzymatique par compétition de l'insuline :

Dans une seconde expérience, la concentration du traceur portant la double mutation D153H/D330N est diminuée d'un facteur 16 de façon à obtenir la même DO à 410 nm de 0,5 après 6 heures de révélation en absence de compétiteur pour le traceur portant les mutations et celui sans mutation. Les expériences sont réalisées dans les mêmes conditions que précédemment et les résultats sont présentés figure 7B. La concentration d'insuline nécessaire pour diminuer de moitié le signal obtenu est 4,5 fois plus faible lorsque l'on utilise le traceur portant les mutations que lorsqu'il s'agit du traceur sans mutation (27 µU et 120 µU respectivement).

Ces résultats montrent que le temps de révélation du dosage est réduit d'un facteur 2 pour le mutant D330N et d'un facteur 6 pour le mutant D153H/D330N et que la sensibilité du dosage (exprimée pour la concentration d'antigène inhibant 50 % du signal) est améliorée d'un facteur 4,5 (cas du double mutant).

**EXEMPLE 11 : Mutagénèse dirigée sur la chimère inactive, au niveau des positions 329 et/ou 330 : sélection des révertants biologiquement actifs.**

En réalisant une mutagénèse dirigée (KUNKEL et al., Methods Enzymol., 1987, **154**, 367-382, précité) sur les positions 329 et 330 respectivement, à partir de la chimère inactive selon l'exemple 1, on obtient les révertants suivants :

D153H/K328H/Q329A/D330H
D153H/K328H/Q329G/D330A
D153H/K328H/Q329G/D330L

qui sont actifs biologiquement et présentent notamment une affinité améliorée pour le substrat enzymatique.

Ainsi que cela ressort de ce qui précède, l'invention ne se limite nullement à ceux de ses modes de mise en oeuvre, de réalisation et d'application qui viennent d'être décrits de façon plus explicite; elle en embrasse au contraire toutes les variantes qui peuvent venir à l'esprit du technicien en la matière, sans s'écarter du cadre, ni de la portée, de la présente invention.

**Revendications**

1. Phosphatase alcaline modifiée d'origine bactérienne, caractérisée en ce qu'elle est constituée par une séquence de phosphatase alcaline bactérienne (BAP), dans laquelle au moins l'un des résidus d'amino-acide en position 329 ou en position 330 est substitué par un autre résidu d'amino-acide, laquelle phosphatase alcaline modifiée présente une activité catalytique et une affinité pour le substrat significativement augmentées par rapport auxdites activités de la phosphatase alcaline bactérienne sauvage correspondante et une stabilité thermique, de l'ordre de celle de ladite phosphatase alcaline bactérienne sauvage.

2. Phosphatase alcaline modifiée d'origine bactérienne selon la revendication 1, caractérisée en ce qu'elle comprend en outre une substitution au niveau du résidu d'amino-acide 153 et/ou du résidu d'amino-acide 328.

3. Phosphatase alcaline modifiée d'origine bactérienne selon la revendication 1 ou la revendication 2, caractérisée en ce que la substitution au niveau de la position 330 est de préférence la substitution d'un acide aspartique (Asp$^{330}$ ou D) par une asparagine (Asn ou N), une alarme (Ala ou A) ou une leucine (Leu ou L).

4. Phosphatase alcaline modifiée d'origine bactérienne selon l'une quelconque des revendications 1 à 3, caractérisée en ce que la substitution au niveau de la position 329 est de préférence la substitution d'une glutamine (Gln$^{329}$ ou Q) par une alanine (Ala ou A).

5. Phosphatase alcaline modifiée d'origine bactérienne selon l'une quelconque des revendications 1 à 4, caractérisée en ce qu'elle comprend en outre une histidine (His ou H) en position 153 à la place d'un acide aspartique (Asp$^{153}$) et/ou une histidine en position 328 à la place d'une lysine (Lys ou K).

6. Phosphatase alcaline modifiée d'origine bactérienne selon l'une quelconque des revendications 1 à 5, caractérisée en qu'elle est sélectionnée dans le groupe qui comprend le mutant D330N, le mutant D153H/D330N, le mutant K328H/D330N et le mutant D153H/K328H/D330N, le mutant D330A, le mutant D330L, le mutant D153H/D330A, le mutant D153H/D330L, le mutant K328H/D330A, le mutant K328H/D330L, le mutant D153H/K328H/D330A, le mutant D153H/K328H/D330L, le mutant Q329A, le mutant D153H/Q329A, le mutant K328H/Q329A, le mutant D153H/K328H/Q329A.

7. Phosphatase alcaline modifiée d'origine bactérienne selon l'une quelconque des revendications 1 à 6, caractérisée en ce que la séquence de phosphatase alcaline bactérienne est de préférence issue d'*Escherichia coli* ou de *Bacillus subtilis*.

8. Phosphatase alcaline modifiée d'origine bactérienne selon l'une quelconque des revendications 1 à 6, caractérisée en ce que la séquence de phosphatase alcaline bactérienne comprend, outre au moins l'une des substitutions telles que définies aux revendications 1 à 6, au moins un amino-acide supplémentaire, inséré entre les amino-acides +6 et +7 de ladite phosphatase alcaline bactérienne.

9. Phosphatase alcaline modifiée d'origine bactérienne selon la revendication 8, caractérisée en ce qu'elle est constituée par la SEQ ID N° 1 ou ses variants.

10. Séquences nucléiques codant pour une phosphatase alcaline selon l'une quelconque des revendications 1 à 9.

11. Plasmide recombinant, caractérisé en ce qu'il comprend une séquence d'acide nucléique codant pour une phosphatase alcaline selon l'une quelconque des revendications 1 à 9 et apte à exprimer ladite phosphatase alcaline dans une cellule hôte convenable.

12. Cellule hôte, transformée par un plasmide selon la revendication 11.

13. Procédé de sélection d'une phosphatase alcaline bactérienne modifiée, possédant à la fois une activité catalytique augmentée par rapport à celle de la phosphatase alcaline bactérienne native et une stabilité thermique de l'ordre de celle de ladite phosphatase alcaline native, caractérisé en ce qu'il comprend :

   i) la préparation d'une phosphatase alcaline chimère inactive comprenant l'introduction dans la séquence codant pour la BAP, d'au moins deux codons codant pour deux résidus d'amino-acides du site actif d'une phosphatase alcaline de mammifère ;
   ii) la réalisation d'une mutagénèse aléatoire ou dirigée sur le gène codant pour cette phosphatase alcaline chimère inactive ;
   iii) l'expression des phosphatase alcalines obtenues en ii) ;
   iv) la sélection des clones bactériens exprimant une phosphatase alcaline dont l'activité enzymatique est restaurée ; et
   v) le séquençage des phosphatases alcalines mutées ainsi obtenues et la sélection de la/les mutations de compatibilité à introduire dans une phosphatase alcaline bactérienne sauvage.

14. Procédé de sélection selon la revendication 13, caractérisé en ce que les mutations introduites à l'étape i) portent au moins sur les résidus d'amino-acide en position 153, 328, 329 et 330 de l'enzyme bactérienne.

15. Procédé de sélection selon la revendication 13 ou la revendication 14, caractérisé en ce que la mutation introduite à l'étape ii) porte au moins sur les résidus d'aminoacides en position 329 et/ou 330 de l'enzyme bactérienne et consiste notamment en la substitution de His en 330 par Asn, Ala ou Leu ou en la substitution en position 329 de Gly par Ala.

16. Procédé de sélection selon l'une quelconque des revendications 13 à 15, caractérisé en ce que les mutations introduites à l'étape i) portent sur l'ensemble des résidus suivants :

   résidu 153 : remplacement de Asp par His,
   résidu 328 : remplacement de Lys par His,
   résidu 329 : remplacement de Gln par Gly,
   résidu 330 : remplacement de Asp par His.

17. Réactif de diagnostic, caractérisé en ce qu'il comprend une phosphatase alcaline selon l'une quelconque des revendications 1 à 9.

18. Phosphatase alcaline chimère constituée d'une phosphatase alcaline bactérienne comprenant au moins 2 résidus d'amino-acide du site actif d'une phosphatase alcaline de mammifère et biologiquement inactive.

**19.** Phosphatase alcaline chimère selon la revendication 18, caractérisée en ce qu'elle correspond au mutant D153H/K328H/Q329G/D330H.

FIGURE 1

Séquence nucléotidique du vecteur pLIP4.0.B : la séquence codante du gène phoA est en gras.

AAGCTTTGGAGATTATCGTCACTGCAATGCTTCGCAATATGGCGCAAAATGACCAACAGCGGTTGATTGATCAGGT

AGAGGGGGCGCTGTACGAGGTAAAGCCCGATGCCAGCATTCCTGACGACGATACGGAGCTGCTGCGCGATTACGTA

AAGAAGTTATTGAAGCATCCTCGTCAGTAAAAAGTTAATCTTTTCAACAGCTGTCATAAAGTTGTCACGGCCGAGA

CTTATAGTCGCTTTGTTTGGATCCTTTAATGTATTTGTACATGGAGAAAATAAAG**TGAAACAAAGCACTATTG**

**CACTGGCACTCTTACCGTTACTGTTTACCCCTGTGACAAAAGCCCGGACACCAGAAATGCCCG**<u>TCGA</u>

<u>CTTCAGTCGACGAGCTCCCGGG</u>**GTTCTGGAAAACCGGGCTGCTCAGGGCGATATTACTGCACCCGGC**

**GGTGCTCGCCGTTTAACGGGTGATCAGACTGCCGCTCTGCGTGATTCTCTTAGCGATAAACCTGCAA**

**AAAATATTATTTTGCTGATTGGCGATGGGATGGGGGACTCGGAAATTACTGCCGCACGTAATTATGC**

**CGAAGGTGCGGGCGGCTTTTTTAAAGGTATAGATGCCTTACCGCTTACCGGGCAATACACTCACTAT**

**GCGCTGAATAAAAAAACCGGCAAACCGGACTACGTCACCGACTCGGCTGCATCAGCAACCGCCTGGT**

**CAACCGGTGTCAAAACCTATAACGGCGCGCTGGGCGTCGATATTCACGAAAAAGATCACCCAACGAT**

**TCTGGAAATGGCAAAAGCCGCAGGTCTGGCGACCGGTAACGTTTCTACCGCAGAGTTGCAGGATGCC**

**ACGCCCGCTGCGCTGGTGGCACATGTGACCTCGCGCAAATGCTACGGTCCGAGCGCGACCAGTGAAA**

**AATGTCCGGGTAACGCTCTGGAAAAAGGCGGAAAAGGATCGATTACCGAACAGCTGCTTAACGCTCG**

**TGCCGACGTTACGCTTGGCGGCGGCGCAAAAACCTTTGCTGAAACGGCAACCGCTGGTGAATGGCAG**

**GGAAAAACGCTGCGTGAACAGGCACAGGCGCGTGGTTATCAGTTGGTGAGCGATGCTGCCTCACTGA**

**ATTCGGTGACGGAAGCGAATCAGCAAAAACCCCTGCTTGGCCTGTTTGCTGACGGCAATATGCCAGT**

**GCGCTGGCTAGGACCGAAAGCAACGTACCATGGCAATATCGATAAGCCCGCAGTCACCTGTACGCCA**

**AATCCGCAACGTAATGACAGTGTACCAACCCTGGCGCAGATGACCGACAAAGCCATTGAATTGTTGA**

**GTAAAAATGAGAAAGGCTTTTTCCTGCAAGTTGAAGGTGCGTCAATCGATAAACAGGATCATGCTGC**

**GAATCCTTGTGGGCAAATTGGCGAGACGGTCGATCTCGATGAAGCCGTACAACGGGCGCTGGAATTC**

**GCTAAAAAGGAGGGTAACACGCTGGTCATAGTCACCGCTGATCACGCCCACGCCAGCCAGATTGTTG**

**CGCCGGATACCAAAGCTCCGGGCCTCACCCAGGCGCTAAATACCAAAGATGGCGCAGTGATGGTGAT**

**GAGTTACGGGAACTCCGAAGAGGATTCACAAGAACATACCGGCAGTCAGTTGCGTATTGCGGCGTAT**

**GGCCCGCATGCCGCCAATGTTGTTGGACTGACCGACCAGACCGATCTCTTCTACACCATGAAAGCCG**

**CTCTGGGGCTGAAATAAAACCGCGCCCGGCAGTGAATTTTCGCTGCCGGGTGGTTTTTTTGCTGTTAGCAACCA**

GACTTAATGGCAGATCACGGGCGCATACGCTCATGGTTAAAAACATGAAGAGGGATGGTGCTATGAAAATAACATTA

CTGGTTACCTTGCTTTTCGGTCTGGTTTTTTTAACCACCGTCGGCGCTGCCGAGAGAACTTTAACCCCACAACAAC

FIGURE 2.1

AGCGTATGACCTCCTGTAATCAGCAGGCGACGGCGCAGGCGTTGAAAGGGGATGCTCGTAAGACCTACATGAGTGA

TTGCCTGAAGAACAGCAAGTCTGCGCCTGGCGAAAAAAGTTTGACGCCACAGCAGCAAAAGATGCGCGAATGCAAT

AATCAAGCAACACAACAATCTCTGAAAGGTGATGATCGTAATAAGTTTATGAGTGCCTGCCTCAAGAAAGCCGCCT

GATACCTGATAGTGCTAACGGGTGAGCTACGAAAATGGCTCACCCGAAATATCATACTTCTGCCTTTAGCTCCGTC

TCTATAATTTGGGAAAATTGTTTCTGAATGTTCCCAAAAATAATGAATGATGAAAACTTTTTCAAAAAAGCGGCGG

CGCACGGGGAGGAACCTCCTTTAACTCCTCAAAACGAACATCAGCGGTCCGGGCTGCGCTTCGCCCGTCGCGTCAG

ACTACCCCGTGCGGTTGGCCTGGCTGGCATGTTCTTACCGATTGCTTCAACGCTGGTTTCACACCCGCCGCCGGGC

TGGTGGTGGCTGGTGTTGGTCGGCTGGGCGTTCGTCTGGCCGCATTTAGCCTGGCAGATAGCGAGCAGGGCCGTCG

ATCCGCTTAGCCGGGAAATTTACAACTTAAAAACCGATGCAGTATTAGCGGGAATGTGGGTAGGCGTAATGGGCGT

AAACGTGCTGCCTTCCACCGCGATGTTGATGATTATGTGTCTGAATTTGATGGGGGCAGGCGGCCCCCGTCTGTTT

GTCGCGGGTCTGGTGTTGATGGTGGTTTCCTGCCTTGTCACCCTCGAGCAAGACGTTTCCCGTTGAATATGGCTCA

TAACACCCCTTGTATTACTGTTTATGTAAGCAGACAGTTTTATTGTTCATGATGATATATTTTTATCTTGTGCAAT

GTAACATCAGAGATTTTGAGACACAACGTGGCTTTGTTGAATAAATCGAACTTTTGCTGAGTTGAAGGATCAGATC

ACGCATCTTCCCGACAACGCAGACCGTTCCGTGGCAAAGCAAAAGTTCAAAATCACCAACTGGTCCACCTACAACA

AAGCTCTCATCAACCGTGGCTCCCTCACTTTCTGGCTGGATGATGGGGCGATTCAGGCCTGGTATGAGTCAGCAAC

ACCTTCTTCACGAGGCAGACCTCAGCGCTAGCGGAGTGTATACTGGCTTACTATGTTGGCACTGATGAGGGTGTCA

GTGAAGTGCTTCATGTGGCAGGAGAAAAAAGGCTGCACCGGTGCGTCAGCAGAATATGTGATACAGGATATATTCC

GCTTCCTCGCTCACTGACTCGCTACGCTCGGTCGTTCGACTGCGGCGAGCGGAAATGGCTTACGAACGGGGCGGAG

ATTTCCTGGAAGATGCCAGGAAGATACTTAACAGGGAAGTGAGAGGGCCGCGGCAAAGCCGTTTTTCCATAGGCTC

CGCCCCCCTGACAAGCATCACGAAATCTGACGCTCAAATCAGTGGTGGCGAAACCCGACAGGACTATAAAGATACC

AGGCGTTTCCCCCTGGCGGCTCCCTCGTGCGCTCTCCTGTTCCTGCCTTTCGGTTTACCGGTGTCATTCCGCTGTT

ATGGCCGCGTTTGTCTCATTCCACGCCTGACACTCAGTTCCGGGTAGGCAGTTCGCTCCAAGCTGGACTGTATGCA

CGAACCCCCCGTTCAGTCCGACCGCTGCGCCTTATCCGGTAACTATCGTCTTGAGTCCAACCCGGAAAGACATGCA

AAAGCACCACTGGCAGCAGCCACTGGTAATTGATTTAGAGGAGTTAGTCTTGAAGTCATGCGCCGGTTAAGGCTAA

ACTGAAAGGACAAGTTTTGGTGACTGCGCTCCTCCAAGCCAGTTACCTCGGTTCAAAGAGTTGGTAGCTCAGAGAA

CCTTCGAAAAACCGCCCTGCAAGGCGGTTTTTTCGTTTTCAGAGCAAGAGATTACGCGCAGACCAAAACGATCTCA

AGAAGATCATCTTATTAAGGGGTCTGACGCTCAGTGGAACGAAAACTCACGTTAAGGGATTTTGGTCATGAGATTA

TCAAAAAGGATCTTCACCTAGATCCTTTTAAATTAAAAATGAAGTTTTAAATCAATCTAAAGTATATATGAGTAAA

CTTGGTCTGACAGTTACCAATGCTTAATCAGTGAGGCACCTATCTCAGCGATCTGTCTATTTCGTTCATCCATAGT

TGCCTGACTCCCCGTCGTGTAGATAACTACGATACGGGAGGGCTTACCATCTGGCCCCAGTGCTGCAATGATACCG

FIGURE 2.2

```
CGAGACCCACGCTCACCGGCTCCAGATTTATCAGCAATAAACCAGCCAGCCGGAAGGGCCGAGCGCAGAAGTGGTC
CTGCAACTTTATCCGCCTCCATCCAGTCTATTAATTGTTGCCGGGAAGCTAGAGTAAGTAGTTCGCCAGTTAATAG
TTTGCGCAACGTTGTTGCCATTGCTGCAGGCATCGTGGTGTCACGCTCGTCGTTTGGTATGGCTTCATTCAGCTCC
GGTTCCCAACGATCAAGGCGAGTTACATGATCCCCCATGTTGTGCAAAAAAGCGGTTAGCTCCTTCGGTCCTCCGA
TCGTTGTCAGAAGTAAGTTGGCCGCAGTGTTATCACTCATGGTTATGGCAGCACTGCATAATTCTCTTACTGTCAT
GCCATCCGTAAGATGCTTTTCTGTGACTGGTGAGTACTCAACCAAGTCATTCTGAGAATAGTGTATGCGGCGACCG
AGTTGCTCTTGCCCGGCGTCAACACGGGATAATACCGCGCCACATAGCAGAACTTTAAAAGTGCTCATCATTGGAA
AACGTTCTTCGGGGCGAAAACTCTCAAGGATCTTACCGCTGTTGAGATCCAGTTCGATGTAACCCACTCGTGCACC
CAACTGATCTTCAGCATCTTTTACTTTCACCAGCGTTTCTGGGTGAGCAAAAACAGGAAGGCAAAATGCCGCAAAA
AAGGGAATAAGGGCGACACGGAAATGTTGAATACTCATACTCTTCCTTTTTCAATATTATTGAAGCATTTATCAGG
GTTATTGTCTCATGAGCGGATACATATTTGAATGTATTTAGAAAAATAAACAAATAGGGGTTCCGCGCACATTTCC
CCGAAAAGTGCCACCTGACGTCTAAGAAACCATTATTATCATGACATTAACCTATAAAAATAGGCGTATGCACGAG
GCCCTTTCGTCTTCAAGAATTTTATAAACCGTGGAGCGGGCAATACTGAGCTGATGAGCAATTTCCGTTGCACCAG
TGCCCTTCTGATGAAGCGTCAGCACGACGTTCCTGTCCACGGTACGCCTGCGGCCAAATTTGATTCCTTTCAGCTT
TGCTTCCTGTCGGCCCTCATTCGTGCGCTCTAGGATCCTCCGGCGTTCAGCCTGTGCCACAGCCGACAGGATGGTG
ACCACCATTTGCCCCATATCACCGTCGGTACTGATCCCGTCGTCAATAAACCGAACCGCTACACCCTGAGCATCAA
ACTCTTTTATCAGTTGGATCATGTCGGCGTGTCGCGGCCAAGACGGTCGAGCTTCTTCACCAGAATGACATCACCT
TCCTCCACCTTCATCCTCAGCAAATCCAGCCCTTCCCGATCTGTTGAACTGCCGGATGCCTTGTCGGTAAAGATGC
GGTTAGCTTTTACCCCTGCATCTTTGAGCGCTGAGGTCTGCCTCGTGAAGAAGGTGTTGCTGACTCATACCAGGCC
TGAATCGCCCCATCATCCAGCCAGAAAGTGAGGGAGCCACGGTTGATGAGAGCTTTGTTGTAGGTGGACCAGTTGG
TGATTTTGAACTTTTGCTTTGCCACGGAACGGTCTGCGTTGTCGGGAAGATGCGTGATCTGATCCTTCAACTCAGC
AAAAGTTCGATTTATTCAACAAAGCCGCCGTCCCGTCAAGTCAGCGTAATGCTCTGCCAGTGTTACAACCAATTAA
CCAATTCTGATTAGAAAAACTCATCGAGCATCAAATGAAACTGCAATTTATTCATATCAGGATTATCAATACCATA
TTTTTGAAAAAGCCGTTTCTGTAATGAAGGAGAAAACTCACCGAGGCAGTTCCATAGGATGGCAAGATCCTGGTAT
CGGTCTGCGATTCCGACTCGTCCAACATCAATACAACCTATTAATTTCCCCTCGTCAAAAATAAGGTTATCAAGTG
AGAAATCACCATGAGTGACGACTGAATCCGGTGAGAATGGCAATTCG
```

## FIGURE 2.3

FIGURE 3

A : Révélation 2 h 30

B : Révélation 24 h

A legend:
● Ea-BAP
□ Ea-BAP D330N
◆ Ea-BAP D153H/D330N

B legend:
● Ea-BAP
□ Ea-BAP D330N

FIGURE 4

FIGURE 5

EP 0 752 475 A1

*tcgac*ttcgttaaccagcacctgtgcgggtcccacctggtggaagctttgtatctggtgtgcggcgagcgtggctt
*g*aagcaattggtcgtggacacgcccagggtggaccaccttcgaaacatagaccacacgccgctcgcaccgaa


cttctacaccccgaagacg**cgtcgtgaagcggaagatctgcaagtgggccaggtggaactgggcggggg**
gaagatgtggggcttctgc**gcagcacttcgccttctagacgttcacccggtccaccttgacccgccccc**


**cccgggcgccggcagcctgcaaccgctggcgctggagggcagcctccagaagcgt**ggcattgtggagc
**gggcccgcggccgtcggacgttggcgaccgcgacctcccgtcggaggtcttcgca**ccgtaacacctcg


agtgttgtactagtatctgcagcctgtaccagctggagaattactgcaac*ggagct*
tcacaacatgatcatagacgtcggacatggtcgacctcttaatgacgttg*cc*


Le peptide C est représenté en gras; les bases ajoutées à la séquence de la proinsuline qui permettent à la fois l'insertion dans le vecteur pLIP5 et la restauration du cadre de lecture du gène *phoA* sont en italiques.


FIGURE 6

FIGURE 7

Office européen
des brevets

**RAPPORT DE RECHERCHE EUROPEENNE**

Numero de la demande

EP 96 40 1419

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | BIOCHEMISTRY, vol. 32, 1993, EASTON, PA US, pages 1601-1609, XP002000142 C.M.L. JANEWAY ET AL: "Magnesium in the active site of Escherichia coli Alkaline phospatase is important for both structural stabilization and catalysis" * le document en entier * --- | 1-7, 10-12, 18,19 | C12N15/55 C12N9/16 C12N1/21 C12N15/10 |
| D,A | WO-A-94 01531 (ABBOT LABORATORIES ) * le document en entier * --- | 1-7, 10-12 | |
| D,A | PROTEIN ENGINEERING, vol. 5, no. 3, 1992, ENGLAND GB, pages 273-278, XP002000143 D. GILLET ET AL: "Insertion of a disulfide-containing neurotoxin into E. coli alkaline phosphatase : the hybrid retains both biological activities" * abrégé; figure 1 * --- | 1,8 | |
| A,D | MOLECULAR MICROBIOLOGY, vol. 12, no. 3, 1994, pages 351-357, XP000566389 J.E. MURPHY ET AL: "Why are mammalian alkaline phosphatases much more active than bacterial alkaline phosphatases" * le document en entier * ----- | 1-7, 10-12, 18,19 | **DOMAINES TECHNIQUES RECHERCHES (Int.Cl.6)** C12N |

Le présent rapport a été établi pour toutes les revendications

| Lieu de la recherche | Date d'achèvement de la recherche | Examinateur |
|---|---|---|
| LA HAYE | 30 Octobre 1996 | Le Cornec, N |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)